(19) Europäisches Patentamt / European Patent Office / Office européen des brevets

(11) EP 4 269 610 B1

(12) **EUROPEAN PATENT SPECIFICATION**

(45) Date of publication and mention of the grant of the patent:
**12.02.2025 Bulletin 2025/07**

(21) Application number: **23181081.3**

(22) Date of filing: **22.06.2018**

(51) International Patent Classification (IPC):
***C12N 15/11** (2006.01)* ***C12Q 1/6809** (2018.01)*
***C12Q 1/6876** (2018.01)* ***C12Q 1/6806** (2018.01)*

(52) Cooperative Patent Classification (CPC):
(C-Sets available)
**C12Q 1/6806; C12N 15/11; C12Q 1/6809; C12Q 1/6876** (Cont.)

# (54) PROBE AND METHOD FOR DETECTING TRANSCRIPT RESULTING FROM FUSION GENE AND/OR EXON SKIPPING

SONDE UND VERFAHREN ZUM NACHWEIS DES TRANSKRIPTS, DER AUS FUSIONSGEN- UND/ODER EXON-SKIPPING RESULTIERT

SONDE ET PROCÉDÉ DE DÉTECTION D’UN PRODUIT DE TRANSCRIPTION RÉSULTANT D’UN GÈNE DE FUSION ET/OU D’UN SAUT D’EXON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2017 JP 2017125074**

(43) Date of publication of application:
**01.11.2023 Bulletin 2023/44**

(62) Document number(s) of the earlier application(s) in accordance with Art. 76 EPC:
**18823127.8 / 3 647 420**

(73) Proprietor: **The University of Tokyo**
**Bunkyo-ku, Tokyo 113-8654 (JP)**

(72) Inventors:
- **MANO, Hiroyuki**
  **Tokyo, 113-8654 (JP)**
- **KOHSAKA, Shinji**
  **Tokyo, 113-8654 (JP)**
- **UENO, Toshihide**
  **Tokyo, 113-8654 (JP)**

(74) Representative: **D Young & Co LLP**
**3 Noble Street**
**London EC2V 7BQ (GB)**

(56) References cited:
**WO-A1-2012/116248 WO-A2-2015/190921**

- **SINGH NATALIA N. ET AL: "A Multi-Exon-Skipping Detection Assay Reveals Surprising Diversity of Splice Isoforms of Spinal Muscular Atrophy Genes", PLOS ONE, vol. 7, no. 11, 19 November 2013 (2013-11-19), pages 1 - 17, XP055897515, Retrieved from the Internet <URL:https://journals.plos.org/plosone/article/file?id=10.1371/journal.pone.0049595&type=printable> DOI: 10.1371/journal.pone.0049595**
- **SRINIVASAN ET AL: "Detection and measurement of alternative splicing using splicing-sensitive microarrays", METHODS, ACADEMIC PRESS, NL, vol. 37, no. 4, 1 December 2005 (2005-12-01), pages 345 - 359, XP005176598, ISSN: 1046-2023, DOI: 10.1016/J.YMETH.2005.09.007**
- **LEVIN JOSHUA Z ET AL: "Targeted next-generation sequencing of a cancer transcriptome enhances detection of sequence variants and novel fusion transcripts", GENOME BIOLOGY, BIOMED CENTRAL LTD, vol. 10, no. 10, 16 October 2009 (2009-10-16), pages R115, XP021065359, ISSN: 1465-6906, DOI: 10.1186/GB-2009-10-10-R115**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6806, C12Q 2535/122, C12Q 2537/159, C12Q 2565/519**

## Description

### Technical Field

The present description relates to a probe for determining the presence or expression level of a transcript of a fusion gene on the genome, a probe for determining the presence of a transcript resulting from exon skipping, a kit containing the probe, a method for determining the presence or expression level of a transcript of a fusion gene on the genome by using the probe, a method for determining the presence or expression level of a transcript resulting from exon skipping, and etc.

### Background Art

A fusion gene is known as a cause of somatic cell mutation. Several therapies have hitherto been developed to overcome a cancer caused by a fusion gene. For example, they include a first line therapy using a tyrosine kinase inhibitor for patients having a carcinogenic mutation such as a BCR-ABL1 fusion gene in chronic myeloid leukemia (non-patent literature 1) and an EML4-ALK fusion gene in non-small cell lung cancer (non-patent literature 2). The treatment results of cancer caused by a fusion gene have been improved owing to the therapy.

Recent technological advancement in sequencing has allowed comprehensive detection of chromosome rearrangement in cancer genome and transcriptome. Consequently, fusion genes such as RET, ROS1, NTRK1, NRG1 and FGRF1/2/3 gene were found (non-patent literatures 3 to 8) and used in diagnosis for cancer. Also, recently, it has been suggested that exon skipping such as MET14 exon skipping may cause cancer in addition to the fusion genes.

However, these fusion genes and exon skipping occur with relatively low frequency and have different variations. Because of this, it was difficult to simultaneously detect a plurality of fusion genes as target genes. Also, conventional methods, such as FISH, immunohistochemistry and reverse transcription-PCR, require technological expertise for use in diagnosis. Therefore, a method of easily detecting a number of target genes is strongly desired for clinical application.

One example of a method for detecting a mutation such as a fusion gene includes target sequencing of a cancer related gene by enrichment of a target gene of gDNA by amplicon PCR or hybridization capture. However, a junction point of, e.g., a fusion gene, is often widely distributed in an intron of each gene. Therefore, an ordinarily used hybridization capture method requires a large number of probes, since it is necessary to prepare probes targeting to the throughout the introns in order to capture the junction point of a fusion gene and exon skipping.

In the meantime, RNA sequencing (RNA-seq) has been proposed as an alternative method for detecting a fusion transcript from a fresh frozen sample or a cell strain. However, RNA-seq is difficult to apply to a sample with law quality of RNA (low-quality RNA sample) such as a formalin fixed paraffin embedded (FFPE) RNA sample, since it is difficult to prepare a reliable library by e.g., poly-A selection usually employed for mRNA concentration. Also, it is reported that a cDNA capture method or anchored multiplex PCR base method is useful as RNA-seq when a low-quality RNA sample is used. However, the type of target gene is extremely limited in these methods, and thus these methods are clinically less useful. Accordingly, a method of easily detecting a large number of target genes even from a low-quality RNA sample has been desired.

WO2012/116248 discloses a method for identifying a tumor as likely to metastasize, or likely to have metastasized, comprising obtaining a sample of the tumor and quantitating alternatively spliced mRNA isoforms of a cell motility gene, a cell adhesion gene and/or an actin cytoskeletal remodelling gene in the sample, or any specified genes or the level of RNA binding proteins compared to a predetermined non-metastasizing control. Levin et al (Genome Biology 2009: 10:R115) discloses that targeted RNA-Seq combines next-generation sequencing with capture of sequences from a relevant subset of a transcriptome.

### Citation List

#### Non Patent Literatures

Non-patent literature 1: J. Erikson et al., Proc. Natl. Acad. Sci., USA 83, 1807-1811, 1986
Non-patent literature 2: M. Soda et al., Nature, 448, 561-566, 2007
Non-patent literature 3: T. Kohno et al., Nat. Med. 18, 375-377, 2012
Non-patent literature 4: K. Takeuchi et al., Nat. Med. 18, 378-381, 2012
Non-patent literature 5: D. Lipson et al., Nat. Med. 18, 382-384, 2012
Non-patent literature 6: L. Fernandez-Cuesta et al., Cancer Discov. 4, 415-422, 2014
Non-patent literature 7: A. Vaishnavi et al., Nat. Med., 19, 1469-1472, 2013

Non-patent literature 8: R. Wang, L et al., Clin. Cancer Res. 20, 4107-4114, 2014

Summary of Invention

Technical Problem

In one embodiment, an object of the present description is to provide a method for easily detecting a transcript resulting from a fusion gene and/or exon skipping.

Solution to Problem

The present inventors have prepared a probe that may be used for detecting a transcript resulting from a fusion gene on the genome or exon skipping in massively parallel sequencing, and have found that the transcript resulting from a fusion gene on the genome or exon skipping can be efficiently detected by using the probe.

The present invention provides a method for determining the presence or expression level of a transcript resulting from exon skipping, the method comprising:

a step of preparing a transcript from a sample derived from a subject;
a step of preparing cDNA from the transcript;
a step of concentrating target cDNA hybridized to a capture probe set;
a step of subjecting the concentrated target cDNA to sequencing by massively parallel sequencing; and
a step of determining the presence or expression level of a transcript resulting from exon skipping; wherein
the transcript comprises exon A' on the 5' side and exon B' on the 3' side linked to each other at a potential junction point,
the capture probe set comprises at least two different capture probes that hybridize to a region derived from either exon A' or B' of cDNA prepared from the transcript, and
$z \geq x + y$ is met, wherein x represents the minimum nucleotide length from an end of each of the capture probes hybridized to the cDNA to the potential junction point, y represents the nucleotide length of the region of each of the capture probes hybridized to cDNA, and z represents the length of a read of massively parallel sequencing.

In a further aspect, the present invention provides a method for determining the presence or absence of a disease or a risk thereof, identifying the type of cancer, or determining prognosis of cancer, in a subject, comprising
a step of determining the presence or expression level of a transcript comprising a transcript resulting from exon skipping in accordance with the method of the present invention.

The description of the present application includes the following aspects.

(1) A probe for determining the presence or expression level of a transcript of a fusion gene on the genome in massively parallel sequencing, wherein

the fusion gene expresses a transcript comprising a part of gene A on the 5' side and a part of gene B on the 3' side linked to each other at a potential junction point,
the probe hybridizes to a region of either gene A or B of cDNA prepared from the transcript, and
$z \geq x + y$ is met where the minimum nucleotide length from an end of the probe hybridized to the cDNA to the potential junction point is represented by x, the nucleotide length of the region of the probe hybridizing to cDNA is represented by y, and the length of a read of massively parallel sequencing is represented by z.

(2) A probe set for determining the presence or expression level of a transcript of a fusion gene on the genome in massively parallel sequencing, wherein

the fusion gene expresses a transcript comprising a part of gene A on the 5' side and a part of gene B on the 3' side linked to each other at a potential junction point,
the probe set comprises at least two different probes that hybridize to a region derived from either gene A or B of cDNA prepared from the transcript, and
$z \geq x + y$ is met where the minimum nucleotide length from an end of each of the probes hybridized to the cDNA to the potential junction point is represented by x, the nucleotide length of the region of each of the probes hybridized to cDNA is represented by y, and the length of a read of massively parallel sequencing is represented by z.

(3) A probe for determining the presence or expression level of a transcript resulting from exon skipping in massively parallel sequencing, wherein

the transcript comprises exon A' on the 5' side and exon B' on the 3' side linked to each other at a potential junction point,
the probe hybridizes to the region derived from either exon A' or B' of cDNA prepared from the transcript, and
$z \geq x + y$ is met where the minimum nucleotide length from an end of the probe hybridized to the cDNA to the potential junction point is represented by x, the nucleotide length of the region of the probe hybridizing to cDNA is represented by y, and the length of a read of massively parallel sequencing is represented by z.

(4) A probe set for determining the presence or expression level of a transcript resulting from exon skipping in massively parallel sequencing, wherein

the transcript comprises exon A' on the 5' side and exon B' on the 3' side linked to each other at a potential junction point,
the probe set comprises at least two different probes that hybridize to a region derived from either exon A' or B' of cDNA prepared from the transcript, and
$z \geq x + y$ is met where the minimum nucleotide length from an end of each of the probes hybridized to the cDNA to the potential junction point is represented by x, the nucleotide length of the region of each of the probes hybridized to cDNA is represented by y, and the length of a read of massively parallel sequencing is represented by z.

(5) The probe or probe set according to any one of (1) to (4), wherein x represents 0 to 140, y represents 30 to 140 and z represents 100 to 300.
(6) The probe set according to any one of (2), (4) and (5), comprising at least 6 said probes.
(7) The probe set according to any one of (2) and (4) to (6), consisting only of probes satisfying $z \geq x + y$.
(8) The probe set according to any one of (2) and (4) to (7), wherein $x_1 = 0$, $x_2 = x_n \times 1/(n-1)$, $x_3 = x_n \times 2/(n-1)$, ...$x_n = x_n \times (n-1)/(n-1)$ where the probe set contains n number of probes, and the minimum nucleotide lengths of individual probes are represented by $x_1$, $x_2$, $x_3$, ... $x_n$ (provided that $x_1 < x_2 < x3 ... < x_n$).
(9) A probe for determining the presence or expression level of a transcript of a fusion gene on the genome in massively parallel sequencing, wherein

the fusion gene expresses a transcript comprising a part of gene A on the 5' side and a part of gene B on the 3' side linked to each other at a potential junction point, and
the probe hybridizes to a region containing the potential junction point of cDNA prepared from the transcript.

(10) A probe set for determining the presence or expression level of a transcript of a fusion gene on the genome in massively parallel sequencing, wherein

the fusion gene expresses a transcript comprising a part of gene A on the 5' side and a part of gene B on the 3' side linked to each other at a potential junction point, and
the probe set comprises at least two different probes that hybridize to a region containing the potential junction point of cDNA prepared from the transcript.

(11) A probe for determining the presence or expression level of a transcript resulting from exon skipping in massively parallel sequencing, wherein

the transcript comprises exon A' on the 5' side and exon B' on the 3' side linked to each other at a potential junction point, and
the probe hybridizes to a region containing a potential junction point, where exon skipping may occur, in cDNA prepared from the transcript.

(12) A probe set for determining the presence or expression level of a transcript resulting from exon skipping in massively parallel sequencing, wherein

the transcript comprises exon A' on the 5' side and exon B' on the 3' side linked to each other at a potential junction point, and
the probe set comprises at least two different probes that hybridize to a region containing the potential junction point, where exon skipping may occur, in cDNA prepared from the transcript.

(13) A combination-probe set comprising a plurality of the probes or the probe sets according to any one of (1) to (12).
(14) The probe or probe set according to any one of (1) to (12) or the combination-probe set according to (13), further comprising at least one probe for determining the expression level.
(15) The probe, probe set or combination-probe set according to any one of (1) to (14), for use for a transcript derived from a processed biological sample.
(16) A kit comprising the probe, probe set or combination-probe set according to any one of (1) to (15).
(17) A method for determining the presence or expression level of a transcript comprising a transcript of a fusion gene on the genome, comprising:

a step of preparing a transcript from a sample derived from a subject;
a step of preparing cDNA from the transcript;
a step of concentrating target cDNA hybridized to the probe, probe set or combination-probe set according to any one of (1) to (15);
a step of subjecting the concentrated target cDNA to sequencing by massively parallel sequencing; and
a step of determining the presence or expression level of a transcript comprising a transcript of a fusion gene on the genome based on the sequencing results.

(18) The method according to (17), wherein the determination is carried out by the following step:

determining that a fusion gene is present, when $0 < \alpha$ or $\beta \leq \gamma$;
determining that a fusion gene is expressed at a low level when $0 < \gamma < \alpha$ or $\beta$; and
determining that a fusion gene is not present when $\alpha$ or $\beta > 0$, $\gamma = 0$;
when the fusion gene expresses a transcript comprising a part of gene A on the 5' side and a part of gene B on the 3' side linked to each other at a potential junction point, and
when the number of reads of cDNA derived from gene A when no gene fusion occurs at a potential junction point is represented by $\alpha$; the number of reads of cDNA derived from gene B is represented by $\beta$; and the number of reads of cDNA derived from a fusion gene when gene fusion occurs at a potential junction point is represented by y.

(19) A method for determining the presence or expression level of a transcript comprising a transcript resulting from exon skipping, comprising:

a step of preparing a transcript from a sample derived from a subject;
a step of preparing cDNA from the transcript;
a step of concentrating target cDNA hybridized to the probe, probe set or combination-probe set according to any one of (1) to (15);
a step of sequencing the concentrated target cDNA concentrated by massively parallel sequencing; and
a step of determining the presence or expression level of a transcript comprising a transcript resulting from exon skipping based on the sequencing results.

(20) The method according to (19), wherein the determination is carried out by the following step:

determining that a transcript resulting from exon skipping is present when $0 < \alpha'$ or $\beta' \leq \gamma'$;
determining that a transcript resulting from exon skipping is expressed at a low level when $0 < \gamma' < \alpha'$ or $\beta'$; and
determining that a transcript resulting from exon skipping is not present when $\alpha'$ or $\beta' > 0$, $\gamma' = 0$;
when the transcript comprises a part of exon A' on the 5' side and a part of exon B' on the 3' side linked to each other at a potential junction point, and when the number of reads of cDNA derived from exon A' when no gene fusion occurs at a potential junction point is represented by $\alpha'$; the number of reads of cDNA derived from exon B' is represented by $\beta'$; and the number of reads of cDNA derived from a transcript resulting from exon skipping is represented by $\gamma'$.

(21) The method according to any one of (17) to (20), wherein the step of determining comprises correcting the expression level of a transcript based on the number of probes, when there is a plurality of probes hybridized to a same region.
(22) The method according to any one of (17) to (21), wherein the step of determining comprises correcting the expression level of a transcript based on the expression level of a housekeeping gene.
(23) A method for determining the presence or absence of onset of a disease or a risk thereof, identifying the type of cancer, or determining prognosis of cancer, in a subject, comprising
a step of determining the presence or expression level of a transcript of a fusion gene on the genome and/or a transcript comprising a transcript resulting from exon skipping in accordance with the method according to any one of (17) to (22).

(24) The method according to (23), wherein the identifying the type of cancer comprises clustering samples derived from subjects based on the presence and/or expression levels of a plurality of transcripts.

The specification contains the disclosure of JP Patent Application No. 2017-125074 to which present application claims priority.

Advantageous Effects of Invention

A method for easily detecting a transcript resulting from a fusion gene and/or exon skipping may be provided by the present description.

Brief Description of Drawings

[Figure 1] Figure 1A is a conceptual diagram of probes according to one embodiment of the present description. In all of the probes shown in the figure, the right end is the 5' end and the left end is the 3' end. The minimum nucleotide length x from an end of the probe to the potential junction point may be determined based on read length z and nucleotide length y of the region of a probe hybridized to cDNA, so that reads supporting junction including a potential junction point may be obtained. Figure 1B shows a method according to an embodiment of the present description, more specifically, a method for detecting a transcript resulting from a fusion gene and/or exon skipping based on the results of sequencing. As shown in Figure 1B, it can be determined that a mutated gene is present when $0 < \alpha$ or $\beta \leq \gamma$; it can be determined that a mutated gene is expressed at a low level when $0 < \gamma < \alpha$ or $\beta$; it can be determined that a mutated gene is not present when $\alpha$ or $\beta > 0$, $\gamma = 0$, where the number of reads of cDNA derived from gene A where no gene mutation (gene fusion or exon skipping) occurs at the potential junction point is represented by $\alpha$, the number of reads of cDNA derived from gene B is represented by $\beta$, and the number of reads of cDNA derived from a fusion gene having a gene mutation at a potential junction point is represented by $\gamma$.

[Figure 2] Figure 2A shows the number of reads supporting junction per 10 M (10 million) raw reads in the indicated methods shown in the figure (Pancancer panel shows all exon capture of synthesized cDNA derived from FFPE sample). The number of probes and the target capture sizes are respectively shown in Figure 2B and Figure 2C when the junction capture method according to an embodiment of the present description and the coding exon capture method known in the art were used. In Figure 2B and 2C, V1, V2, and V3 show the results of gene panels (TOP RNA V1, TOP RNA V2, and TOP RNA V3) described in Examples.

[Figure 3] Figure 3A shows the mapping results of sequence reads to a MET transcript, in MET exon 14 skipping (+) cases, by RNA-seq in accordance with 3 different methods: i.e., poly A selection (poly A capture) of RNA extracted from a fresh frozen sample; all exon capture (Pancancer panel) of synthesized cDNA derived from an FFPE sample; or junction capture of synthesized cDNA derived from an FFPE sample. In the figure, the region flanked by two vertical lines shows the region corresponding to MET exon 14. No reads present in this region means exon skipping positive. Figure 3B shows the number of reads supporting junction (exon skipping) per 10 million raw read of MET exon 13 and MET exon 15 in the indicated methods.

[Figure 4] Figure 4A shows a representative photograph of a bone marrow aspirate specimen stained with hematoxylin and eosin (200 × magnification, scale bar 100 μm). Figure 4B shows representative photographs of TBLB specimens stained with hematoxylin and eosin s (left, 40 × magnification, scale bar 1 mm; right, 400 × magnification, scale bar 100 μm).

[Figure 5] Figure 5 is a graph showing the correlation between RPKM of RNA-seq and RPKM corrected based on the tiling number in the junction capture method. The results of measurement of expression level of a group of genes are shown in A; whereas the results of analysis of fusion gene of a group of genes are shown in B. Correlation was observed in all 7 samples.

[Figure 6] Figure 6 shows the results of clustering of samples based on the expression level of genes. The vertical axis represents individual genes. Clustering was carried out based on intensity of expression. The horizontal axis represents individual samples. It is found that samples were clustered in accordance with the types of cancers such as LUAD, SARC, MUCA, and LUSC.

Description of Embodiments

1. Probe for determining the presence or expression level of a transcript of a fusion gene on the genome

In one aspect, the present description relates to a probe for determining the presence or expression level of a transcript of a fusion gene on the genome in massively parallel sequencing.

The "massively parallel sequencing" as used herein refers to a method of sequencing DNA massively in parallel. In the massively parallel sequence, usually, $10^2$, $10^3$, $10^4$, $10^5$ or more DNA molecules are simultaneously sequenced. Massively parallel sequencing incudes, for example, a next-generation sequencing.

The next-generation sequencing is a method for obtaining sequence information using a next-generation sequencer and characterized in that an enormous number of sequencing reactions can be carried out simultaneously in parallel compared with the Sanger method (see, for example, Rick Kamps et al., Int. J. Mol. Sci., 2017, 18 (2), p. 308 and Int. Neurourol. J., 2016, 20 (Suppl. 2), S76-83). Various systems are provided for the next generation sequencing. Examples of the systems that can be used include, but are not limited to, Genome Sequencer (GS) FLX System (Roche), HiSeq or Genome Analyzer (GA) (Illumina), Support Oligonucleotide Ligation Detection (SOLiD) system (Life technologies), G.007 system (Polonator) and HeliScope Gene Sequencing system (Helicos BioSciences).

Although it is not limited, typical steps of the next generation sequencing are shown below. In the next generation sequencing, at first, a sample is prepared. In this step, a nucleic acid to be analyzed is enzymatically or mechanically fragmented depending on the read length of the next-generation sequencer. Subsequently, an adapter sequence required for the following sequencing step is often added. A specific gene region may be enriched by, e.g., PCR, or concentrated by using, e.g., a probe having a specific sequence to analyze the specific gene region. The gene region may be enriched by an amplification step comprising, for exapmle, 4 to 12 cyclic operations. The concentration using a probe may be conducted by using a label (for example, biotin) attached to a probe.

Subsequently, sequencing is carried out. The detailed operation of this step varies depending on the type of next-generation sequencer. Typically, a nucleic acid is bound to a substrate via an adaptor sequence, and then, a sequencing reaction is carried out by using the adapter sequence as a priming site. Regarding details of the sequence reaction, see, for example, Rick Kamps et al. (supra).

Finally, data are output. In this step, a collection of sequence information (reads) obtained by the sequencing reaction can be obtained. Output data are further analyzed to obtain more meaningful results such as the number of reads, for example, the number of reads supporting junction per raw read.

The "number of reads" as used herein refers to the amount of an amplified product having a specific sequence. Since the number of reads usually increases in proportional to the amount of nucleic acid before sequencing, the expression level of a gene can be estimated based on the number of reads.

The "read supporting junction" as used herein refers to a read containing a junction point in a transcription product resulting from gene fusion or exon skipping, or a junction point on the genome resulting from gene fusion or exon skipping. The "read supporting junction number" means the number of reads supporting junction. The "raw read" as used herein refers to the total number of reads obtained by the next generation sequencing. The frequency of appearance of the read supporting junction can be evaluated by calculating the number of reads supporting junction per raw read.

The "fusion gene on the genome" as used herein refers to a mutated gene resulting from linking a plurality of genes as the result of chromosome rearrangement resulting from, e.g., deletion, insertion, inversion and translocation. Usually, a fusion gene is transcribed to produce an RNA molecule as an expression product. Examples of the RNA molecule include a transcript such as mRNA encoding a fusion protein. Examples of the fusion gene as used herein include, but are not limited to, fusion genes having carcinogenicity, such as EML4-ALK, BCR-ABL1, KIF5B-RET, SLC34A2-ROS1, CD74-ROS1, SS18-SSX1, SS18-SSX2, NAB2-STAT6, EWSR1-FLI1, SYT-SSX1, FUS-CREB3L2, TPM3-ROS1, CD74-NRG1 and EWSR1-FLI1.

In the present description, the "presence" of a transcript of a fusion gene on the genome refers to the presence or absence of the fusion gene on the genome. The "expression level" of a transcript of a fusion gene refers to the expression level of a transcript such as mRNA, rRNA and tRNA derived from a fusion gene and preferably the expression level of mRNA.

In one embodiment, the probe of the present description hybridizes to a region derived from either gene A or B of cDNA prepared from a transcript when a fusion gene expresses a transcript comprising a part of gene A on the 5' side and a part of gene B on the 3' side linked to each other at a potential junction point. Individual genes that can form a fusion gene, and the potential junction point may be determined with reference to scientific papers, patent literatures and database such as COSMIC.

The "exon" as used herein refers to a region remaining in the nucleotide sequence of a mature transcript, among the nucleotide sequence of a gene. Generally in eukaryotes, a mature transcript is formed by removing intervening regions called introns from the primary transcript by splicing after a gene is transcribed as a primary transcript and linking exons to each other. For example, a mature miRNA arises, which is constituted by exons linked to each other, removing introns by pre-miRNA splicing from mRNA precursor (pre-miRNA) produced by transcription, in the case of a gene encoding a protein.

In one embodiment, the probe is designed so as to satisfy $z \geq x + y$ when the minimum nucleotide length from either the end on the 5' side or the end on the 3' side of each probe to the potential junction point is represented by x when the probe is hybridized to cDNA prepared from a transcript of an RNA molecule; the nucleotide length of the region of the probe hybridized to cDNA is represented by y; and the length of a read in massively parallel sequencing probe is represented by z. The probe that hybridizes to the nucleic acid region not containing a potential junction point will be hereinafter referred to also as the "potential junction point non-covering probe". The potential junction point non-covering probe has an advantage of being able to detect a plurality of fusion partners and new fusion genes.

To facilitate understanding of the present invention, the design of the probe according to the embodiment is shown in Figure 1A. Figure 1A shows that the minimum nucleotide length from an end of the probe to a potential junction point is represented by x, the nucleotide length of the region of the probe hybridized to cDNA is represented by y, and the length of a read is represented by z, and that a read containing a potential junction point can be obtained by massively parallel sequencing.

In one embodiment, read length z is determined by an apparatus and a method employed in massively parallel sequencing. If a nucleic acid derived from a sample is already fragmented and/or if a nucleic acid is fragmented before sequencing, the read length may be determined by the lengths of these fragments. The read length z may be, but not limited to, for example, 50 or more, 75 or more, 100 or more, 150 or more or 160 or more; and 500 or less, 400 or less, 300 or less, 200 or less or 180 or less; for example, may be 50 to 500, 100 to 300 or 150 to 200. Note that, massively parallel sequencing includes single-end sequencing, which is sequencing carried out only from one end of a nucleic acid, and paired-end sequencing, which is sequencing carried out from both ends of a nucleic acid. The above read length z is preferably a read length obtained by the paired-end sequencing.

The nucleotide length y of a probe hybridized to cDNA can be appropriately determined by those skilled in the art. The length y may be, for example, 20 or more, 30 or more, 40 or more, and preferably 50 or more, 60 or more or 80 or more; and 220 or less, 200 or less, 180 or less, and preferably 160 or less, 140 or less or 120 or less; for example, 20 to 220, 50 to 160 or 60 to 140. Preferably, a probe hybridizes to cDNA in the continuous region from the end near a potential junction point. In one embodiment, a probe hybridizes to cDNA over its entire sequence. In this case, the value y is equal to the length of the probe.

The nucleotide length of a probe may be, but not limited to, for example, 20 or more, 40 or more, 60 or more, 80 or more, 100 or more, 110 or more or 115 or more; and 220 or less, 200 or less, 180 or less, 160 or less, 140 or less, 130 or less or 125 or less; for example, 20 to 220, 60 to 180, 100 to 140, 110 to 130, 115 to 125 or 120.

The minimum nucleotide length x from an end of the probe to a potential junction point can be appropriately determined based on the read length z and the nucleotide length y of the region of a probe hybridized to cDNA. For example, the lower limit of the minimum nucleotide length x (from an end of the probe to a potential junction point) is 0, which meansthat the probe is designed so as to hybridize to the region adjacent to a potential junction point. The upper limit of the length x may be, but not limited to, for example, 300 or less, 250 or less, 200 or less, 150 or less, 140 or less, 130 or less, 125 or less, or 120 or less. For example, the length x may be 0 to 300, 0 to 200, 0 to 140, 0 to 125 or 0 to 120.

The expression of $z \geq x + y + a$ ($a \geq 0$) indicates that a read having a sequence comprising nucleotide length "a" beyond a potential junction point. If a plurality of probes targeting to the region near a potential junction point are designed in this manner, various types of transcripts of a fusion gene can be efficiently concentrated by using these probes. The value "a" is not particularly limited as long as it is 0 or more. The value "a" can be appropriately determined by those skilled in the art with reference to the disclosure of the specification, taking into consideration that the value "a" is large, the specificity increases but detection sensitivity decreases. The value of "a" is, for example, 5 or more, 10 or more and preferably 15 or more, 20 or more, 30 or more, 50 or more or 100 or more; and 500 or less, 400 or less, and preferably 300 or less, 200 or less or 150 or less.

A probe can be easily designed by those skilled in the art based on the sequence of a target gene. The "target gene" as used herein refers to a gene that may be captured by the probe of the present description; for example, a gene that may form a fusion gene and a gene in which exon skipping may occur.

Example of such a probe include a probe comprising any of the following nucleotide sequences:(a) a nucleotide sequence comprising at least consecutive 20, 40, 60, 80, 100, 110, 115 or 120 nucleotides complementary to a target gene; (b) a nucleotide sequence having the nucleotide sequence (a) in which one or more nucleotides are added, deleted, and/or substituted; (c) a nucleotide sequence having an identity of, for example, 70% or more, 80% or more, preferably 90% or more, 95% or more, 97% or more, 98% or more or 99% or more with the nucleotide sequence (a); and (d) the nucleotide sequence of a nucleic acid hybridizing with a target gene having at least consecutive 20, 40, 60, 80, 100, 110, 115 or 120 nucleotides under stringent conditions.

The range of "one or more" as used herein is 1 to 10, preferably 1 to 7, further preferably 1 to 5, and particularly preferably 1 to 3, or 1 or 2. The value showing an identity with a nucleotide sequence as used herein is a value calculated by the software that calculates the identity between a plurality of sequences (for example, FASTA, DANASYS and BLAST) with the default setting. Regarding a method to detect identity , for example, see, Altschul et al, Nuc. Acids. Res. 25, 3389-3402, 1977 and Altschul et al, J. Mol. Biol. 215, 403-410, 1990.

The "stringent conditions" as used herein refer to conditions under which a so-called specific hybrid is formed and a non-specific hybrid is not formed. As the stringent conditions, the conditions of a hybridization method known in the art can be employed. The stringent conditions may be appropriately determined referring to, for example, Green and Sambrook, Molecular Cloning, 4th Ed (2012), Cold Spring Harbor Laboratory Press. More specifically, stringent conditions may be set by specifying the temperature of a hybridization process, the concentration of a salt contained in a solution; and the temperature of a washing step of the hybridization process and the concentration of a salt contained in the washing solution. More specific stringent conditions include, for example, a sodium concentration of 25 to 500 mM and preferably 25 to 300 mM, the temperature of 42 to 68°C and preferably 42 to 65°C. More specifically, the conditions include 5 × SSC (83 mM NaCl, 83 mM sodium citrate) and a temperature of 42°C.

The probe can be prepared based on the aforementioned sequence by the method known to those skilled in the art including, but not limited to, chemical synthesis.

In one embodiment, the present description relates to a probe set containing at least two different probes as mentioned above. The number of probes is not particularly limited as long as the number is 2 or more. If the number of probes is extremely low, the detection sensitivity decreases; whereas, if the number of probes is extremely large, the cost increases. Therefore, the number of probes may be appropriately determined with reference to the disclosure of the specification in consideration of, e.g., sensitivity and cost. The number of probes which may be contained in the probe set may be, for example, 3 or more, 4 or more, 5 or more, 6 or more, 8 or more, 10 or more or 11 or more, and 30 or less, 25 or less, 20 or less, 15 or less, 14 or less, 13 or less or 12 or less.

The values of minimum nucleotide length x from an end of a probe to a potential junction point of the each probe contained in the probe set are preferably not equal and vary. This allows various nucleic acid fragments to be captured. For example, the minimum nucleotide length values of the probes can be determined so as to satisfy the following expression:

[Expression 1]

$$x_1=0\pm b,\ x_2=x_n\times 1/(n-1)\pm b,\ x_3=x_n\times 2/(n-1)\pm b,\ \ldots x_n=x_n\times (n-1)/(n-1)\pm b$$

when a probe set contains n number of probes and the minimum nucleotide length values of the probes are defined as $x_1$, $x_2$, $x_3$, ...$x_n$ (provided that, $x_1 < x_2 <$ x3... $< x_n$). In the expression, b represents a constant. If b represents 0, it means that the minimum nucleotide length values x of the probes are distributed at constant intervals from a potential junction point. As the value of b increases, the distribution from the potential junction point becomes more uneven. The value b is, for example 50 or less, 40 or less, 30 or less, 25 or less, 20 or less, 15 or less, 10 or less, preferably 5 or less, 4 or less, 3 or less, 2 or less, 1 or less or 0. The value $x_n$ may be any value, for example, 20 to 500, 30 to 400, 40 to 300, 60 to 200, 80 to 180, preferably100 to 140, 110 to 130, 115 to 125 or 120.

If the number n of probes is 3 or more, after probes are designed in accordance with the above expression, m number of probes may be removed from a set of the probes (provided that, m represents an integer of 1 or more, for example, 1 to 5, 1 to 4, 1 to 3, 1 to 2, and preferably 1; and $n-m \geq 2$).

In one embodiment, the probe of the present description can be used for concentrating a specific nucleic acid sequence before a sequencing step of the next generation sequencing.

In one aspect, the probe of the present description hybridizes to a nucleic acid region comprising a potential junction point. The probe that hybridizes to a nucleic acid region comprising a potential junction point will be hereinafter referred to a "potential junction point covering probe". Constitutions of the potential junction point covering probe or a set thereof are the same as the constitutions of the "potential junction point non-covering probe" described above (for example, the nucleotide length y of a probe that hybridizes to cDNA and the number of probes contained in the probe set) other than that they comprise a probe hybridizes to the nucleic acid region comprising a potential junction point. However, the potential junction point covering probe detects only single fusion gene resulting from the fusion a part of gene A and a part of gene B, and thus the potential junction point covering probe has a high specificity but cannot detect various fusion partners.

In one embodiment, a potential junction point covering probe hybridizes to nucleotides of 10 nucleotides or more, 15 nucleotides or more, 20 nucleotides or more, 30 nucleotides or more, 40 nucleotides or more, 50 nucleotides or more or 60 nucleotides or more of the region derived from both of gene A on the 5' side and gene B on the 3' side of cDNA, which is prepared from a transcript of the fusion gene.

In one embodiment, the probe set of the present description comprises "potential junction point covering probe" in addition to the "potential junction point non-covering probe". Detection specificity may be further enhanced when comprising both types of probes. In one embodiment, the probe set of the present description only consists of a probe satisfying $z \geq x + y$ and a potential junction point covering probe. In another embodiment, the probe set of the present description only consists of a probe satisfying $z \geq x + y$.

The probe set of the present description may be designed for regions on 5' end side and 3' end side of all exons of a target gene to be evaluated. It is preferable to design probes for only regions on the 5' end side and/or 3' end side of the exon involved in gene fusion of a gene known to form a fusion gene.

In one embodiment, the probe of the present description or probe set further comprises at least one probe for measuring gene expression level. The probe for measuring gene expression level refers to a probe used for measuring gene expression level in massively parallel sequencing. The probe for measuring gene expression level can be designed evenly for all genes whose expression level is measured, more specifically, at a density of 2 × tiling or more. The nucleotide length of the probe for measuring gene expression level may be, but not limited to, 20 or more, 40 or more, 60 or more, 80 or more, 100 or more, 110 or more or 115 or more; and 220 or less, 200 or less, 180 or less, 160 or less, 140 or less, 130 or less or 125 or less; and for example, 20 to 220, 60 to 180, 100 to 140, 110 to 130, 115 to 125 or 120. The number of the probes for measuring gene expression level per gene may be, but not limited to, for example, 3 or more, 4 or more, 5 or more, 6 or more, 8 or more, 10 or more or 11 or more; and 30 or less, 25 or less, 20 or less, 15 or less, 14 or less, 13 or less or 12 or less. The probes for measuring gene expression level may be probes for a plurality of genes, for example, 2 or more, 5 or more, 10 or more, 50 or more, 100 or more, 150 or more, 200 or more, 250 or more, preferably, 300 or more, 400 or more or 500 or more; and 2000 or less, 1000 or less, 900 or less, preferably, 800 or less, 700 or less or 600 or less. Examples of the target genes whose expression level are measured include oncogenes (for example, ALK, EGFR, ERBB2, MET) and housekeeping genes. A nucleic acid that can hybridize to at least a part of these genes can be used as a probe. The gene expression level can be more accurately measured when comprising the probe for measuring gene expression level.

In one embodiment, the present description relates to a combination probe set including a plurality of different probes or probe sets described above. The combination probe set preferably includes a set of probes that hybridize to a plurality of different fusion genes. This allows the simultaneous detection of the presence of transcripts of a plurality of fusion genes and expression levels thereof. The lower and upper limits of the "plurality" may be, but not particularly limited to, 2 or more, 5 or more, 10 or more, 50 or more, 100 or more, 150 or more, 200 or more, 250 or more, preferably 300 or more, 400 or more or 500 or more; and 2000 or less, 1000 or less, 900 or less, preferably 800 or less, 700 or less or 600 or less.

In one embodiment, the probe, probe set or combination-probe set as described herein is preferably applied to a transcript derived from a sample containing decomposed or deteriorated RNA such as a processed biological sample. The examples of the processing include heat treatment, freezing treatment, acid treatment and treatment with a base. Preferably, fixation such as FFPE (formalin fixed paraffin embedding) is mentioned.

2. Effect of the probe of the present description

As mentioned above, The probe of the present description can capture and concentrate a nucleic acid fragment from which a read covering a potential junction point can be obtained by massively parallel sequencing, and thus a fusion gene may be efficiently detected by subjecting the concentrated sample to massively parallel sequencing. In one embodiment, the probe set of the present description is used for cDNA prepared from a transcript such as mRNA and may contain probes intensively targeting to the region near a potential junction point. Therefore, the probe set may have an advantage that the number of requisite probes is low compared to an intron capture method (capturing introns of genome DNA) and a coding-exon capture method (capturing all exons). In one embodiment, various types of nucleic acid fragments containing a potential junction point can be obtained, since the probe set of the present description contains probes intensively targeting to the region near a potential junction point. It was surprising that the detection efficiency of a fusion gene or exon skipping is improved when containing probes intensively targeting to the region near a potential junction point, since Ryan Tewhey et al. (Genome Biology, 2009, 10, R116) report that coverage is not improved by increasing a density of a probe to 2 × tiling or more. The "tiling" as used herein refers to a density of probes designed to hybridize to a target gene, the multiple number n of tiling means that probes are designed at intervals of w/n where the length of the probe is represented by w.

In one embodiment, a fusion gene can be efficiently detected, in particular, in a sample containing decomposed or deteriorated RNA, since the probe of the present description does not require a poly A sequence contained in mRNA for transcription or concentration.

3. Probe for determining the presence or expression level of a transcript resulting from exon skipping

In one aspect, the present description relates to a probe for determining the presence or expression level of a transcript resulting from exon skipping in massively parallel sequencing or a probe set containing at least 2 different said probes. The probe of the aspect hybridizes to the region derived from either exon A' or B' of cDNA prepared from the transcript, when the transcript comprises Exon A' on the 5' side and exon B' on the 3' side linked to each other at a potential junction point. In one embodiment, $z \geq x + y$ is met when each probe hybridizes to cDNA prepared from the transcript, the minimum nucleotide length from an end of the probe to the potential junction point is represented by x, the nucleotide length of the region of each probe that hybridizes to cDNA is represented by y, and length of the read obtained by massively parallel sequencing is represented by z.

In one aspect, the present description relates to a probe for determining the presence or expression level of a transcript resulting from exon skipping in massively parallel sequencing, wherein exon A' on the 5' side and exon B' on the 3' side are linked to each other at a potential junction point in the transcript, and wherein the probe hybridizing to the region containing the potential junction point where exon skipping may occur in cDNA prepared from the transcript; or relates to a probe set containing at least two probes different said probes.

The "exon skipping" as used herein refers to a phenomenon where abnormal joining of exons occurs as a result of removal of a part of exons by splicing error in addition to introns. One example is a case where exon B' is skipped out by splicing error so that exon A' and exon C' are linked if a wild type gene contains exon A', B' and C', and exon A', B' and C' are linked in a normal case. The resultant of exon skipping is an abnormal product, and thus often causes a disease. For example, it is known that skipping of MET (mesenchymal-epithelial transition) exon 14 is related to a morbidity of non-small cell lung cancer.

The constitutions of the probe of the aspect other than relating to a method for determining the presence or expression level of a transcript resulting from exon skipping, for example, the number of probes, minimum nucleotide length x from an end of each probe to a potential junction point, nucleotide length y of the region of each probe hybridized to cDNA, length z of the read in massively parallel sequencing, sequence of each probe and design thereof, are the same as defined in the above section "1. Probe for determining the presence or expression level of a transcript of a fusion gene on the genome". The probe of the aspect may further comprise a gene expression measurement probe, in the same way as the "1. Probe for determining the presence or expression level of a transcript of a fusion gene on the genome". The effect of the probe of the aspect is the same as the above "2. Effect of the probe of the present description ".

In one aspect, the present description relates to a probe set containing both the "Probe for determining the presence or expression level of a transcript resulting from exon skipping" of this aspect and the above "1. Probe for determining the presence or expression level of a transcript of a fusion gene on the genome". Both a fusion gene and exon skipping can be simultaneously detected by using this probe set.

4. Kit containing probe

In one aspect, the present description relates to a kit containing the probe, probe set, or combination probe set comprising the above "1. Probe for determining the presence or expression level of a transcript of a fusion gene on the genome" and/or the above "3. Probe for determining the presence or expression level of a transcript resulting from exon skipping".

The kit may comprise e.g., a buffer, an enzyme and instructions, in addition to the probe as described above.

The kit may be used for determining the presence or expression level of a transcript of a fusion gene and/or for determining the presence or expression level of a transcript resulting from exon skipping.

5. Method for determining the presence or expression level of a transcript comprising a transcript of gene a fusion

In one aspect, the present description relates to a method for determining the presence or expression level of a transcript comprising a transcript of a fusion gene on the genome. The method according to the aspect comprises, in this order: a step of preparing a transcript from a sample derived from a subject (transcript preparation step); a step of preparing cDNA from the transcript (cDNA preparation step); a step of concentrating a target cDNA hybridized to a probe according to the probe, probe set or a combination-probe set of the "1.Probe for determining the presence or expression level of a transcript of a fusion gene on the genome" (concentration step); a step of sequencing the target cDNA concentrated by massively parallel sequencing (sequencing step); and a step of determining the presence or expression level of a transcript of a fusion gene on the genome based on the sequencing results (determination step).

Individual steps constituting the method are more specifically described hereinbelow.

(1) Transcript preparation step

In the transcript preparation step, a transcript is prepared from a sample derived from a subject. The biological species of the subject as used herein is, but not limited to, preferably mammal, for example a primate such as a human and a chimpanzee; an experimental animal such as a rat and a mouse; a livestock animal such as a pig, a cow, a horse, a sheep and a goat; or a pet animal such as a dog and a cat, and preferably a human.

The "sample" as used herein refers to a biological sample to be subjected to the method of the present invention. Examples of the sample that can be used in the present invention include, but are not limited to, a body fluid, cells and tissues isolated, for example, from a living body. Examples of the body fluid include blood, sweat, saliva, milk and urea.

Examples of the cells include peripheral blood cells, lymph and tissue-fluid containing cells, hair matrix cells, oral cells, nasal cells, intestinal cells, vaginal cells, mucosal cells and sputum (that may comprise e.g., alveolar cells or pneumocytes). Examples of the tissue include cancer lesions, for example, brain, pharynx, thyroid, lung, breast, esophagus, stomach, liver, pancreas, kidney, small intestine, colon, bladder, prostate, uterus and ovary, and preferably lung. More specifically, biopsy samples of these tissues may be used. The pathological symptoms of a subject can be more accurately specified when a biopsy sample is used, since a histopathological diagnosis and detection of a fusion gene according to the present description can be simultaneously carried out.

In one embodiment, as a sample, a sample containing decomposed or deteriorated RNA, for example, a processed biological sample is used. The examples of the processing include heat treatment, freezing treatment, acid treatment and treatment with a base, and preferably, fixing such as FFPE (formalin fixed paraffin embedding).

Examples of a transcript (total RNA) include rRNA, tRNA and mRNA, and preferably mRNA.

A transcript is prepared from a sample using a method known in the art. For example, a transcript may be extracted by mixing a sample with a lysate containing guanidine thiocyanate and a surfactant, applying physical processing (e.g., stirring, homogenizing, ultrasonic crushing) to the obtained mixture. Preferably, the AGPC method may be employed, which is a method of further adding phenol and chloroform to a sample, stirring and centrifuging the sample and recovering an aqueous phase containing a transcript. Subsequently, a transcript may be obtained from the aqueous phase by alcohol precipitation. RNA may be extracted using a commercially available kit such as RNA-Bee (Tel-Test Inc.) and TRIZOL (Thermo Fisher Scientific). For specific procedures of these methods, see, protocols in the art, for example, Green and Sambrook, Molecular Cloning, 4th Ed (2012), Cold Spring Harbor Laboratory Press. Regarding other biological methods as described herein, for example, the following cDNA preparation step and concentration step, see, Green and Sambrook (supra).

(2) cDNA preparation step

cDNA may be prepared by a reverse transcription reaction from the transcript obtained in the transcript preparation step, using a reverse transcriptase. The primers, reverse transcriptase and reaction conditions to be employed in the reverse transcription reaction can be appropriately selected from those known in the art by those skilled in the art. In the method of the present invention, it is not necessary to subject mRNA alone to reverse transcription using poly A sequence, and for example, total RNA may be subjected to reverse transcription using, for example, random primers, since nucleic acid fragments of interest is concentrated in the concentration step described below.

(3) Concentration step

In the concentration step, target cDNA hybridized to the probe, probe set or combination-probe set described herein is concentrated. The concentration may be carried out using a method known to those skilled in the art. More specifically, a probe may be tagged with a label, and a target cDNA hybridized to the probe may be concentrated by the interaction between the label and another substance. For example, cDNA hybridized to the probe tagged with biotin may be concentrated by the interaction with avidin. cDNA may be concentrated by affinity chromatography using a substrate or an antigen-antibody reaction. Alternatively, magnetic beads may be attached to a probe and cDNA hybridized to the probe may be concentrated by magnetic force.

Before and after the concentration step by the probe set, cDNA may be enzymatically or mechanically fragmented depending on the length of a read in massively parallel sequencing. Further, an adaptor sequence required in the sequencing step described later may be added. A specific gene region may be amplified by, e.g., PCR, in order to analyze the specific gene region before or after the concentration step. The gene region may be enriched by an amplification step comprising, for example, 4 to 12 cycles.

(4) Sequencing step

In the sequencing step, the concentrated target cDNA is sequenced by massively parallel sequencing. The details of the sequencing step vary depending on, for example, the apparatus to be used in massively parallel sequencing. Typically, target cDNA is bound to a substrate via an adaptor sequence, and then, a sequencing reaction is carried out by using the adapter sequence as a priming site. Regarding details of the sequence reaction, see, for example, Rick Kamps et al. (supra).

In this step, a collection of sequence information (reads) obtained by the sequencing reaction can be obtained. Output data are further analyzed to obtain more meaningful results such as the number of reads, for example, the number of reads supporting junction per raw read. The apparatus for use in massively parallel sequencing is commercially available. Examples of the apparatus that can be used include, but are not limited to, Genome Sequencer (GS)FLX System (Roche), HiSeq or Genome Analyzer (GA) (Illumina), Support Oligonucleotide Ligation Detection (SOLiD) system (Life technologies), G.007 system (Polonator) and HeliScope Gene Sequencing system(Helicos BioSciences).

(5) Determination step

In the determination step, the presence or expression level of a transcript of a fusion gene on the genome is determined based on the results of the sequencing step. An example of the determination step is shown in Figure 1B. The specific process of the determination step may be conducted, but not limited to, for example, based on the following criteria:

determining that a fusion gene is present, when $0 < \alpha$ or $\beta \leq \gamma$;
determining that a fusion gene is expressed at a low level when $0 < y < \alpha$ or $\beta$; and
determining that a fusion gene is not present when $\alpha$ or $\beta > 0$, $y = 0$;

When the fusion gene expresses a transcript comprising a part of gene A on the 5' side and a part of gene B on the 3' side linked to each other at a potential junction point, and when the number of reads of cDNA derived from gene A when no fusion occurs at the potential junction point is represented by $\alpha$, the number of reads of cDNA derived from gene B in this case is represented by $\beta$, and the number of reads of cDNA derived from a fusion gene in which gene fusion occurs at the potential junction point is represented by y.

It is believed that the transcript of a fusion gene is not present or the transcript is decomposed due to low quality of the sample, when $\alpha$ and/or $\beta = 0$ and $y = 0$. In this case, it can be accurately determined which one of the cases is correct by precisely counting the number of reads of wild-type transcripts of the two genes constituting a potential fusion gene near the potential junction point.

The number of reads increases usually in proportion to the amount of nucleic acid before sequencing. Thus, the expression level can be determined based on the number of reads. The expression level can be determined based on a relative value obtained by comparing the read number with that of wild-type genes or comparing the read number with that of a healthy subject or determined based on an absolute value, i.e., measured value, of e.g., the number of reads obtained in a specific condition.

In one embodiment, the determination step comprises, when a plurality of probes that hybridize to the same region, are present. Since the probe set of the present description contains probes intensively targeting to the region near a potential junction point, redundant probes targeting to the same region may be designed. Due to this, the number of reads of transcripts corresponding to the region may be increased in calculation. Thus, the number of reads is preferably corrected based on the number of probes that hybridize to the same region, in order to more accurately determine the expression level based on the number of reads. The method for correcting the number of reads based on the number of probes is not limited. For example, the number of reads may be corrected by dividing the number of reads by tiling number of probes (for example, the number of reads may be divided by 5 when $5 \times$ tiling, and the number of reads can be divided by 10 when $10 \times$ tiling).

In one embodiment, the determination step comprises correcting the expression level of a transcript based on the expression level of at least one housekeeping gene. Correction based on the housekeeping gene is particularly preferable when expression level is more accurately compared in the case where different probe sets are used and/or where different samples are used. The housekeeping genes known in the art can be used. For example, at least one, at least two, at least three, at least five or all of ACTB, B2M, GAPDH, GUSB, H3F3A, HPRT1, HSP90AB1, NPM1, PPIA, RPLPO, TFRC and UBC may be used. Although the method of correcting the number of reads by a housekeeping gene(s) is not limited, for example, the number of reads can be corrected by dividing the number of reads of a transcript whose expression level is to be measured by the number of reads of housekeeping gene(s).

A disease may be diagnosed by determining the presence or expression level of a fusion gene on the genome by the method of the aspect. Also, a therapy such as an appropriate drug, may be selected in view of the genetic background of a subject including the presence or expression level of a fusion gene on the genome.

6. Method for determining the presence or expression level of a transcript comprising a transcript resulting from exon skipping

In one aspect, the present invention relates to a method for determining the presence or expression level of a transcript comprising a transcript resulting from exon skipping. The method of the aspect comprises, in this order: a step of preparing a transcript from a sample derived from a subject (transcript preparation step); a step of preparing cDNA from the transcript (cDNA preparation step); a step of concentrating a target cDNA hybridized to a probe, a probe set or a combination-probe set described in the above "3. Probe for determining the presence or expression level of a transcript resulting from exon skipping" (concentration step); a step of sequencing the target cDNA concentrated by massively parallel sequencing (sequencing step); and a step of determining the presence or expression level of a transcript comprising a transcript resulting from exon skipping based on the sequencing results (determination step).

The constitutions of the method according to the aspect other than relating to determining the presence or expression level of a transcript comprising a transcript resulting from exon skipping and using different probes, for example, the transcript preparation step, cDNA preparation step, concentration step, sequencing step and determination step, are the same as defined in the above section "5. Method for determining the presence or expression level of a transcript of a fusion gene". Accordingly, the differences from the above section "5. Method for determining the presence or expression level of a transcript of a fusion gene" are mainly described hereinbelow.

In one aspect, the present invention relates to a method comprising a cDNA concentration step, using both of the above "1.Probe for determining the presence or expression level of a transcript of a fusion gene on the genome" and the above "3. Probe for determining the presence or expression level of a transcript resulting from exon skipping". Both of a fusion gene and exon skipping can be simultaneously detected by this embodiment.

The determination step may be carried out as described in the above "5. Method for determining the presence or expression level of a transcript of a fusion gene". More specifically, The determination step may be carried out by,

determining that a transcript resulting from exon skipping is present when $0 < \alpha'$ or $\beta' \leq \gamma'$;
determining that a transcript resulting from exon skipping is expressed at a low level when $0 < \gamma' < \alpha'$ or $\beta'$; and
determining that a transcript resulting from exon skipping is not present when $\alpha'$ or $\beta' > 0$, $\gamma' = 0$;

when the transcript comprises a part of gene A on the 5' side and a part of gene B on the 3' side linked to each other at a potential junction point, and when the number of reads of cDNA derived from exon A' when no gene fusion occurs at a potential junction point is represented by $\alpha'$, the number of reads of cDNA derived from exon B' is represented by $\beta'$ and the number of reads of cDNA derived from a transcript resulting from exon skipping is represented by $\gamma'$.

7. Method for determining the presence or absence of a disease or a risk thereof, identifying the type of cancer, or determining prognosis of cancer

In one aspect, the present description relates to a method for determining the presence or absence of a disease or a risk thereof, identifying the type of cancer (for example, primary cancer), or determining prognosis of cancer (or cancer patients), the method comprising a step of determining the presence or expression level of a transcript of a fusion gene on the genome and/or a transcript comprising a transcript resulting from exon skipping in accordance with the method as described herein (determination step). The determination step may be carried out in the same manner as described in, the above section "5. Method for determining the presence or expression level of a transcript of a fusion gene" and/or the above section "6. Method for determining the presence or expression level of a transcript comprising a transcript resulting from exon skipping". The method of the aspect differs from the method described in the above section "5. Method for determining the presence or expression level of a transcript of a fusion gene" or "6. Method for determining the presence or expression level of a transcript comprising a transcript resulting from exon skipping", in relating to determining the presence or absence of a disease or a risk thereof, identifying the type of cancer or determining prognosis of cancer.

In the method of the aspect, the type of disease is not limited as long as the presence or absence of a disease or a risk thereof can be determined by a fusion gene or exon skipping. Examples of the disease include brain tumor, pharyngeal cancer, thyroid cancer, lung cancer, breast cancer, esophageal cancer, stomach cancer, liver cancer, pancreatic cancer, kidney cancer, small intestine cancer, colorectal cancer, bladder cancer, prostate cancer, cervical cancer, ovarian cancer, sarcoma, lymphoma and melanoma, preferably, lung cancer or sarcoma.

The method of the aspect, in addition to the determination step, may comprise: a step of evaluating the presence or absence of a disease or a risk thereof (evaluation step); a step of identifying the type of cancer (identification step); or a step of determining prognosis of cancer (determination step), in a subject based on the presence or expression level of a transcript of a fusion gene on the genome and/or the presence or expression level of a transcript resulting from exon skipping.

Evaluation step

The evaluation step may be carried out by using the relationship between a fusion gene or exon skipping and a disease, known in the art. For example, the presence or absence of the diseases or risk thereof can be determined by EML4 (echinoderm microtubule associated protein like 4)-ALK (Anaplastic lymphoma kinase) for non-small cell lung cancer; BCR (B cell receptor)-ABL1 (Abelson murine leukemia viral oncogene homolog 1) for chronic myeloid leukemia, TAF15 (TATA-box binding protein associated factor 15)-NR4A3 (nuclear receptor subfamily 4 group A member 3) for extraosseous chondrosarcoma, AHRR (aryl-hydrocarbon receptor repressor)-NCOA2 (nuclear receptor coactivator 2) for hemangiofibroma, and MET-exon 14 skipping for non-small cell lung cancer.

In the evaluation step, it can be evaluated that a subject is affected with the disease or has a high risk of onset thereof when the presence of a transcript of a fusion gene or a transcript resulting from exon skipping is detected or when the expression level of a fusion gene or the expression level of a transcript resulting from exon skipping is high compared to that, for example, of a healthy subject.

Identification step and determination step

Identification of the type of cancer and determination of prognosis of cancer can be carried out by using the relationship between a transcript of a fusion gene on the genome and/or a transcript comprising a transcript resulting from exon skipping and a disease. As the relationship between the transcript and a disease, that known in the art may be used and that unknown in the art may be used.

The "prognosis" as used herein refers to reduction of tumor volume, suppression of tumor growth, progression of a disease or ending (for example, relapse or not, survival or death), preferably, the length of lifetime or high or low risk for recurrence, for example, after applying a therapeutic treatment such as chemotherapy. The determination of prognosis may be prediction of, for example, survival time or the survival rate after a certain period of time, for example, after applying a therapeutic treatment.

In one embodiment, the identification and determination steps comprise clustering samples derived from subjects based on the presence of a plurality of transcripts and/or expression levels thereof. This embodiment is particularly advantageous in the case where the relationship between the transcript and the disease is unknown. The number of transcripts in this embodiment may be, but not limited to, for example, 2 or more, 5 or more, 10 or more, 20 or more, 30 or more, 50 or more, 100 or more, 200 or more, 300 or more, 400 or more or 500 or more; and 20000 or less, 10000 or less, 5000 or less, preferably 3000 or less, 2000 or less or 1000 or less. A reference sample derived from a subject having a specified type of cancer or prognosis previously determined may be added when clustering samples based on the presence of a plurality of transcripts and/or expression levels thereof. Due to this, it is possible to more accurately cluster the samples based on the type of cancer or prognosis thereof. The method of clustering may be, but not limited to, for example, a method of clustering samples based on gene expression levels using statistical analysis software R, heatmap.3.

In the identification step, the type of cancer include, but not limited to, brain tumor, pharyngeal cancer, thyroid cancer, lung cancer (for example, lung adenocarcinoma), breast cancer, esophageal cancer, stomach cancer, liver cancer, pancreatic cancer, kidney cancer, small intestine cancer, colorectal cancer, bladder cancer, prostate cancer, cervical cancer, ovarian cancer, sarcoma, lymphoma or melanoma, preferably lung cancer (for example, lung adenocarcinoma) or sarcoma.

A method of determining the presence or absence of a disease or a risk thereof, identifying the type of cancer, or determining the prognosis of cancer according to the aspect may be conducted in combination with other methods, for example, histopathological diagnosis, detection of a biomarker by e.g., FISH, RT-PCR and immunohistochemistry, and diagnostic imaging such as CT, MRI and nuclear medicine examination. The detection accuracy of a disease can be enhanced by combining with other methods.

## Examples

### Materials and methods

#### gDNA target sequencing

Genomic DNA (500 ng) was isolated from an FFPE sample by GeneRead DNA FFPE Kit (Qiagen) and a target fragment was enriched by using SureSelectXT Custom Kit (Agilent). Custom-made probes were designed so as to hybridize to and capture gDNA of a target gene. The fragments were isolated and subjected to massively parallel sequencing using HiSeq2500 platform (Illumina) in accordance with paired end sequencing. Only sequencing reads of Q value ≥ 20 for each nucleotide were selected from a large dataset, and mapped on reference human genome sequencing (hg19) by using bowtie 2 algorithm (http://bowtie-bio.sourceforge.net/bowtie2/index.shtml). Somatic mutation was identified by MuTect (http://www.broadinstitute.org/cancer/cga/mutect). Mutation candidates were selected based on the following determination criteria: judgement = KEEP (KEEP represents somatic mutation-positive by mutect), tumor read depth ≥ 20 ×, mutation rate ≥ 10% and normal read depth ≥ 10 ×.

#### RNA-seq by poly A selection

Total RNA was extracted from a fresh frozen sample using RNA-Bee (Tel-Test Inc., # CS-104B), treated with DNase I (Life Technology) and subjected to poly A-RNA selection, and the resultant was used for cDNA synthesis. RNA-seq library was prepared by using NEBNext Ultra Directional RNA Library Prep Kit (New England Bio Labs) in accordance with the protocol of the manufacturer. NGS sequencing was conducted from both ends of each cluster by using HiSeq2500 platform (Illumina).

RNA-seq by cDNA capture

Total RNA was extracted from an FFPE sample by using RNeasy FFPE Kit (Qiagen), and treated with DNase I (Life Technology). Synthesis of cDNA and capturing with a probe, and preparation of a library for a coding exon capture method by using TruSight RNA Pan-Cancer Panel (Illumina) in accordance with the manufacturer's protocol.

Synthesis of cDNA and library preparation for a junction capture method were carried out by using SureSelect RNA Capture kit (Agilent technologies) in accordance with the manufacturer's protocol. The custom probe for the junction capture method was designed such that the probe hybridizes to the sequence near potential junction point of a target gene and captures it. More specifically, probes were designed such that the minimum nucleotide length from an end of each of probes when hybridized to cDNA to the potential junction point is 120 or less, taking into consideration that the length of a read in used massive parallel sequencing is 170 bp, and assuming that a read covering a potential junction point can be obtained when the nucleotide length of the region of a probe hybridizing to cDNA is 50 or more. The lengths of probes were all set at 120 bp. In the junction capture method, probes were designed at a 5 × or 10 × tiling to obtain various reads as many as possible. NGS sequencing was carried out by using HiSeq2500 platform (Illumina) from both ends of each cluster. Table 1 below shows SEQ ID numbers of probe sets used for identifying exon 13 of EML4, exon 20 of ALK and a fusion gene of EML4-ALK as representatives.

[Table 1]

| | SEQ ID NO: |
|---|---|
| EML 4(exon 13) | 1 |
| EML 4(exon 13) probe 1 | 2 |
| EML 4(exon 13) probe 2 | 3 |
| EML 4(exon 13) probe 3 | 4 |
| EML 4(exon 13) probe 4 | 5 |
| EML 4(exon 13) probe 5 | 6 |
| ALK(exon 20) | 7 |
| ALK(exon 20) probe 1 | 8 |
| ALK(exon 20) probe 2 | 9 |
| ALK(exon 20) probe 3 | 10 |
| ALK(exon 20) probe 4 | 11 |
| ALK(exon 20) probe 5 | 12 |

Example 1: Detection of fusion gene by junction capture method

Results

In analysis for sequence data, the number of sequence reads which supports the presence of a junction point of a fusion transcript was counted, and it was investigated whether the fusion transcript is significantly expressed or not in comparison with the transcripts of wild type genes.

It is shown that the fusion transcript is not present when transcripts of genes are present and a transcript of a fusion gene is not present. When the number of reads of each of the genes is 0, it was carefully evaluated whether this is caused by no expression of mRNA or by decomposition of mRNA due to the quality of a sample.

A small target panel (TOP RNA V1) targeting 67 fusion genes based on the junction capture method was prepared as a pilot experiment. TOP RNA V1 panel was compared to the panel obtained by a conventional method, i.e., an intron capture method (TOP DNA) detecting a junction point on the genome of a fusion gene or TruSight RNA Pan-Cancer Panel (illumina) based on a coding exon capture method.

As a result, the TOP RNA V1 panel obtained by the junction capture method can detect fusion genes more accurately, and the values of reads supporting junction/10 million raw reads were larger than the TOP DNA panel obtained by the intron capture method (Table 2, Figure 2A). The results suggest that the junction capture method is a superior method for detecting a fusion gene.

[Table 2]

| Case # | Diagnosis | RIN score | Fusion gene | Junction capture | | gDNA capture |
|---|---|---|---|---|---|---|
| | | | | Number of reads supporting junctions | Version | Number of reads supporting junctions |
| #1 | NSCLC | - | EML4-ALK | 232 | V1 | 12 |
| #2 | NSCLC | - | EML4-ALK | 284 | V1 | 108 |
| #3 | NSCLC | - | EML4-ALK | 180 | V1 | 44 |
| #4 | NSCLC | - | EML4-ALK | 4179 | V1 | 0 |
| #5 | NSCLC | - | KIF5B-RET | 514 | V1 | 0 |
| #6 | NSCLC | - | KIF5B-RET | 2898 | V1 | 150 |
| #7 | NSCLC | 1.3 | KIF5B-RET | 1189 | V1 | 53 |
| #8 | NSCLC | - | SLC34A2-ROS1 | 1633 | V1 | 17 |
| #9 | NSCLC | 1.4 | CD74-ROS1 | 5268 | V1 | 0 |
| #10 | NSCLC | - | CD74-ROS1 | 2491 | V1 | 77 |
| #11 | SS | - | SS18-SSX1 | 4410 | V2 | NA |
| #12 | SS | 1.1 | SS18-SSX2 | 492 | V2 | NA |
| #13 | SS | - | SS18-SSX1 | 629 | V2 | NA |
| #14 | SS | 2 | NAB2-STAT6 | 4232 | V2 | NA |
| #15 | SS | 2 | EWSR1-FLI1 | 110 | V2 | NA |
| #16 | SS | 2.2 | SYT-SSX1 | 1446 | V2 | NA |
| #17 | LGFS | 1.4 | FUS-CREB3L2 | 213 | V2 | NA |
| #18 | NSCLC | 1 | EML4-ALK | 109 | V3 | NA |
| #19 | NSCLC | - | EML4-ALK | 119 | V3 | NA |
| #20 | NSCLC | 1.9 | EML4-ALK | 175 | V3 | NA |
| #21 | NSCLC | 1.8 | EML4-ALK | 156 | V3 | NA |
| #22 | NSCLC | 1.7 | EML4-ALK | 396 | V3 | NA |
| #23 | NSCLC | - | EML4-ALK | 107 | V3 | NA |
| #24 | NSCLC | - | KIF5B-RET | 369 | V3 | NA |
| #25 | NSCLC | - | KIF5B-RET | 423 | V3 | NA |
| #26 | NSCLC | - | CD74-ROS1 | 13 | V3 | NA |
| #27 | NSCLC | - | CD74-ROS1 | 28 | V3 | NA |
| #28 | NSCLC | 1.9 | TPM3-ROS1 | 32 | V3 | NA |
| #29 | NSCLC | 1.3 | CD74-NRG1 | 59 | V3 | NA |
| #30 | ARSM | - | EWSR1-FLI1 | 35 | V3 | NA |
| #31 | EWS | 1 | EWSR1-FLI1 | 412 | V3 | NA |
| #32 | EWS | 1.6 | EWSR1-FLI1 | 544 | V3 | NA |
| #33 | EWS | 2.2 | EWSR1-FLI1 | 394 | V3 | NA |

In the Table, NSCLS represents non-small cell lung cancer; SS represents synovial sarcoma; LGFS represents low-grade fibromyxoid sarcoma, ARSM represents alveolar rhabdomysarcoma and EWS represents Ewing sarcoma.

Subsequently, a larger target panel (TOP RNA V2) covering a sarcoma fusion gene and a panel covering all fusion genes reported in the database COSMIC (TOP RNA V3) were designed, with respect to the junction capture method. The RNA integrity score (RIN) of stored FFPE samples, from which RNA was extracted, were 1.1 to 2.3, which shows that decomposition highly proceeds. However, all fusion transcripts were detected (Table 3). Further, the number of probes estimated and the sizes of the target sequences captured (length of a nucleic acid captured by probes) were both significantly low in the panel designed by the junction capture method, compared to the panel designed by the coding exon capture method(Figure 2B and Figure 2C). This suggests that the junction capture method is highly cost-effective.

The quality of RNA-seq can be evaluated by calculating the coverage and cover ratios of housekeeping genes. The quality of RNA-seq is determined as being excellent based on the following criteria: average cover ratio of housekeeping gene > 500X and 100X and cover ratio of housekeeping gene > 70%. When the read supporting junction is not present, there is a possibility that the read supporting junction is not detected because decomposition of RNA derived from FFRE proceeds. Then, a pipeline for counting the number of reads supporting junction of wild type transcripts of the two genes constituting a putative fusion gene reported in COSMIC database, was developed, in order to confirm that a fusion gene is really negative. Based on the results of analysis for Case #31 (EML4-ALK positive lung adenocarcinoma), it was confirmed that the tumor is really negative for a fusion transcript analyzed (data not shown).

Example 2: Detection of exon skipping by junction capture method

Subsequently, it was investigated whether the junction capture method can detect a transcript resulting from, e.g., MET exon 14 skipping reported to be carcinogenic in lung adenocarcinoma. RNA was extracted from 5 FFPE samples of lung adenocarcinoma cases, which were specified to have MET exon 14 skipping by RNA-seq using fresh frozen samples. The number of reads supporting junction, which support junction between exon 13 and exon 15, in other words, skipping of exon 14, was counted. In all 5 FFPE samples having exon skipping, MET exon 14 skipping was identified by the junction capture method, and no reads supporting junction were found in other 34 cases having no MET exon skipping (Figure 3, Table 3). This suggests that exon skipping is also detectable by the junction capture method.

[Table 3]

| | Fresh frozen sample | FFPE | |
|---|---|---|---|
| Case # | Poly A capture | Pancancer panel | Junction capture |
| #35 | 1689 | 828 | 27918 |
| #36 | 310 | 256 | 12182 |
| #37 | 1226 | 242 | 25607 |
| #38 | 431 | 272 | 3528 |
| #39 | 84 | 348 | 5076 |
| #1-34 | NA | 0 | 0 |

Example 3: Application of junction capture method to biopsy sample

Whether the junction capture method can be applied to small biopsy samples was evaluated. RNA samples were prepared from fusion-gene positive FFPE specimens including core needle bipsy, fine needle aspiration biopsy and transbronchial lung biopsy (TBLB). Surprisingly, a large number of reads supporting junction supporting correct fusion transcripts specific to each specimen were detected, in all of RNA-seq (Figure 4, Table 4).

[Table 4]

| Case # | Diagnosis | Sampling date | RIN | Fusion gene | Number of reads supporting junctions | Number of raw reads | TOP version | Biopsy |
|---|---|---|---|---|---|---|---|---|
| #40 | ARSM | 2011 | - | PAX3-FOXO1 | 237 | 35,522,326 | V2 | Core needle biopsy |
| #41 | ARSM | 2010 | 1.3 | PAX7-FOXO1 | 1499 | 31,848,250 | V2 | Fine needle aspiration biopsy |
| #42 | EWS | 2015 | 1.3 | EWSR1-FLI1 | 362 | 31,372,328 | V3 | Core needle biopsy |

(continued)

| Case # | Diagnosis | Sampling date | RIN | Fusion gene | Number of reads supporting junctions | Number of raw reads | TOP version | Biopsy |
|---|---|---|---|---|---|---|---|---|
| #43 | NSCLC | 2014 | NA | EML4-ALK | 142 | 36,012,196 | V3 | TBLB |

Example 4: Clinical usefulness of junction capture method

Clinical usefulness of the method was evaluated by examining KRAS and EGFR mutation-negative FFPE samples obtained by surgical excision of 40 cases of stage II or III NSCLC by the junction capture method. 3 cases of MET exon 14 skipping, 2 cases of EML4-ALK fusion gene and 1 case of RET fusion gene were detected (data not shown). The junction capture method was applied to sarcoma patients in a prospective study to evaluate clinical usefulness of the junction capture method for diagnosing sarcoma. The results are shown in the following Table 5.

[Table 5]

| Case # | Site | Excision date | Diagnosis with excised sample | Fusion gene detected by TOP-RNA | Final diagnosis |
|---|---|---|---|---|---|
| #44 | Left knee | 2017/2/8 | Myxofibrosarcoma | AHRR-NCOA2 | Soft-tissue hemangiofibroma |
| #48 | Left thigh | 2017/4/12 | Extraosseous chondro-sarcoma | TAF15-NR4A3 | Extraosseous chondro-sarcoma |

One case (#44) was diagnosed as myxofibrosarcoma due to growth of spindle cells having an atypical nucleus near the mucous stroma. However, this case was determined as soft-tissue hemangiofibroma, since a fusion gene, i.e., AHRR-NCO2A gene, specific to hemangiofibroma was detected by the junction capture method. Another case (#48) is TAF15-NR4A3 positive, which is consistent with diagnosis results as extraosseous chondrosarcoma.

These results suggest that the junction capture method can be used for diagnosis for a disease.

Example 5: Measurement of gene expression level

Gene expression levels were measured by junction capture method in the example.

(Materials and methods)

Gene expression measurement

Total RNA was extracted from an FFPE sample in accordance with Example 1 and RNA-seq was carried out by cDNA capture (junction capture) for 11 housekeeping genes (ACTB, B2M, GAPDH, GUSB, H3F3A, HPRT1, HSP90AB1, PPIA, RPLPO, TFRC and UBC) in accordance with Example 1. Total RNA was extracted from a fresh frozen sample in accordance with Example 1 and RNA-Seq was carried out by poly A selection, for comparison.

In this example, a probe for measuring gene expression level was added to carry out concentration, in addition to a custom probe (TOP RNA V3) for the junction capture method shown in Example 1. As the probe for measuring the gene expression level, probes for 125 genes including an oncogene such as ERBB2, which were designed at a density of 2 × tiling, were used. The lengths of the probes were all set at 120 bases.

Correction of number of reads based on the tiling number

**[0113]** As described in Example 1, probes were designed to intensively target to the region near potential junction point at a density of 5 × or 10 × tiling, in order to obtain various reads as many as possible by the junction capture method. Accordingly, when estimating the expression levels of genes based on the number of reads, there is a risk that the expression levels may increase depending on the number of probes in calculation. Then, the number of reads was corrected by dividing the number of reads by tiling number of probes in the junction capture method (for example, the number of reads was divided by 5 when the density was 5 × tiling, and the number of reads was divided by 10 when the density was 10 × tiling).

Correction of number of reads based on housekeeping gene

Difference of quality between the samples was corrected such that the expression levels of housekeeping genes become equal, since FFPE samples were used in the junction capture method (Group A), whereas the fresh frozen samples were used in RNA-Seq using poly A selection (Group B). More specifically, the coefficient for correcting the expression levels of Group B was calculated such that log_2 average of the ratio of expression levels of 11-types of housekeeping genes between Group A and Group B becomes equal. Using these coefficients, expression levels of all genes were corrected.

(Results)

Expression levels of 11 types of housekeeping genes (ACTB, B2M, GAPDH, GUSB, H3F3A, HPRT1, HSP90AB1, NPM1, PPIA, RPLPO, TFRC and UBC) were measured by RNA-Seq using poly A selection and the junction capture method, with respect to 7 samples derived from lung cancer patients.

As a result, it was confirmed that there is a correlation between RPKM (Reads Per Kilobase of exon model per Million mapped reads) values in RNA-Seq using poly A selection and the junction capture method (data not shown), with respect to the housekeeping genes.

Subsequently, the correlation coefficient between a gene group for measuring expression level and a gene group for analyzing a fusion gene was calculated, with respect to RPKM of RNA-seq and RPKM corrected based on the tiling number in the junction capture method,. The gene group for measuring expression level refers to a gene group whose expression level was measured by probes for measuring gene expression level. The gene group for analyzing a fusion gene refers to a gene group whose expression level was measured by a custom probe by the junction capture method.

The results of the gene group for measuring expression level are shown in Figure 5A and Table 6. The results of the gene group for analyzing a fusion gene are shown in Figure 5B and Table 7. It was confirmed that there is a correlation between the RPKM of RNA-seq and RPKM of the junction capture method, in both of the gene group for measuring expression level and the gene group for analyzing a fusion gene, in particular, a strong correlation in the gene group for measuring expression level was found. These results show that although probes for measuring gene expression level are more suitable for measurement of expression level, custom probes for the junction capture method can be used for measurement of expression level. Also, these results show that even if custom probes for the junction capture method are contained in addition to probes for measuring gene expression level, gene expression level can be accurately measured.

[Table 6]

| sample | Correlation coefficient |
|---|---|
| Sample-1 | 0.938599 |
| Sample-2 | 0.971988 |
| Sample-3 | 0.962161 |
| Sample-4 | 0.953048 |
| Sample-5 | 0.991559 |
| Sample-6 | 0.990007 |
| Sample-7 | 0.99219 |

[Table 7]

| sample | Correlation coefficient |
|---|---|
| Sample-1 | 0.817235 |
| Sample-2 | 0.770109 |
| Sample-3 | 0.860437 |
| Sample-4 | 0.782432 |
| Sample-5 | 0.822337 |
| Sample-6 | 0.630832 |

(continued)

| sample | Correlation coefficient |
|---|---|
| Sample-7 | 0.801661 |

Example 6: Clustering of cancer based on gene expression level

Samples derived from patients with LUAD (lung adenocarcinoma), SARC (sarcoma), MUCA (multiple cancer) and LUSC (lung squamous cell carcinoma) were subjected to measurement of gene expression by the junction capture method using probes for measuring gene expression level in addition to the custom probes, in accordance with Example 5. More specifically, expression levels of total 467 genes including both genes used in measurement of expression level and analysis of fusion gene were measured after correction of the number of reads based on the tiling number and correction of the number of reads based on housekeeping genes in accordance with Example 5. The obtained expression levels (xn, n = 1, ...,N, N represents the number of genes) were subjected to logarithmic conversion (log_2 (xn + 1)). Clustering was carried out using statistical analysis software R, heatmap.3, based on the logarithmic values.

As a result, clustering of LUAD, SARC, MUCA and LUSC was carried out based on the expression levels of genes, as shown in Figure 6. This demonstrates that the types of primary cancers can be specified by measuring gene expression levels by the method of the present invention.

Industrial Applicability

A method for easily detecting a transcript resulting from a fusion gene and/or exon skipping can be provided by the present description. It becomes possible to diagnose a disease and select an appropriate drug in consideration of genetic background of a subject by the invention, and thus industrial applicability of the invention is high.

## Claims

1. A method for determining the presence or expression level of a transcript resulting from exon skipping, the method comprising:

   a step of preparing a transcript from a sample derived from a subject;
   a step of preparing cDNA from the transcript;
   a step of concentrating target cDNA hybridized to a capture probe set;
   a step of subjecting the concentrated target cDNA to sequencing by massively parallel sequencing; and
   a step of determining the presence or expression level of a transcript resulting from exon skipping; wherein the transcript comprises exon A' on the 5' side and exon B' on the 3' side linked to each other at a potential junction point,
   the capture probe set comprises at least two different capture probes that hybridize to a region derived from either exon A' or B' of cDNA prepared from the transcript, and
   $z \geq x + y$ is met, wherein x represents the minimum nucleotide length from an end of each of the capture probes hybridized to the cDNA to the potential junction point, y represents the nucleotide length of the region of each of the capture probes hybridized to cDNA, and z represents the length of a read of massively parallel sequencing.

2. The method according to claim 1, wherein z is 50 to 500.

3. The method according to claim 1, wherein x represents 0 to 140, y represents 30 to 140 and z represents 100 to 300.

4. The method according to any one of claims 1 to 3 wherein the capture probe set is a combination-probe set comprising the capture probe sets defined in any one of claims 1 to 3.

5. The method according to any one of claims 1 to 4 wherein the capture probe set as defined in any one of claims 1 to 3 or the combination-probe set as defined in claim 4 further comprises at least one probe for measuring gene expression level.

6. A method for determining the presence or absence of a disease or a risk thereof, identifying the type of cancer, or determining prognosis of cancer, in a subject, comprising
   a step of determining the presence or expression level of a transcript comprising a transcript resulting from exon skipping in accordance with the method according to any one of claims 1 to 5.

## Patentansprüche

1. Verfahren zur Bestimmung des Vorliegens oder Expressionsniveaus eines Transkripts, das aus Exon-Skipping resultiert, wobei das Verfahren Folgendes umfasst:

   einen Schritt, bei dem ein Transkript aus einer von einem Individuum stammenden Probe hergestellt wird;
   einen Schritt, bei dem cDNA aus dem Transkript hergestellt wird;
   einen Schritt, bei dem an einen Einfangsondensatz hybridisierte Ziel-cDNA konzentriert wird;
   einen Schritt, bei dem die konzentrierte Ziel-cDNA massiv paralleler Sequenzierung unterzogen wird; und
   einen Schritt, bei dem das Vorliegen oder Expressionsniveau eines Transkripts, das aus Exon-Skipping resultiert, bestimmt wird; wobei
   das Transkript Exon A' auf der 5'-Seite und Exon B' auf der 3'-Seite umfasst, die an einem potentiellen Verbindungspunkt miteinander verknüpft sind,
   der Einfangsondensatz wenigstens zwei verschiedene Einfangsonden umfasst, die an eine Region hybridisieren, die von entweder Exon A' oder B' von aus dem Transkript hergestellter cDNA stammt, und
   $z \geq x + y$ gilt, wobei x für die minimale Nukleotidlänge von jeweils einem Ende der an die cDNA hybridisierten Einfangsonden bis zum potentiellen Verbindungspunkt steht, y für die Nukleotidlänge der Region jeder der an cDNA hybridisierten Einfangsonden steht und z für die Länge eines Read von massiv paralleler Sequenzierung steht.

2. Verfahren nach Anspruch 1, wobei z 50 bis 500 beträgt.

3. Verfahren nach Anspruch 1, wobei x für 0 bis 140, y für 30 bis 140 und z für 100 bis 300 steht.

4. Verfahren nach einem der Ansprüche 1 bis 3, wobei der Einfangsondensatz ein Kombinationssondensatz ist, der die Einfangsondensätze gemäß einem der Ansprüche 1 bis 3 umfasst.

5. Verfahren nach einem der Ansprüche 1 bis 4, wobei der Einfangsondensatz gemäß einem der Ansprüche 1 bis 3 oder der Kombinationssondensatz gemäß Anspruch 4 ferner wenigstens eine Sonde zum Messen des Genexpressionsniveaus umfasst.

6. Verfahren zur Bestimmung des Vorliegens oder Fehlens einer Krankheit oder eines Risikos davon, Identifizierung der Krebsart oder Bestimmung einer Krebsprognose bei einem Individuum, umfassend
einen Schritt, bei dem das Vorliegen oder Expressionsniveau eines Transkripts, das ein aus Exon-Skipping resultierendes Transkript umfasst, entsprechend dem Verfahren nach einem der Ansprüche 1 bis 5 bestimmt wird.

## Revendications

1. Procédé pour déterminer la présence ou le niveau d'expression d'un transcrit résultant d'un saut d'exon, le procédé comprenant :

   une étape de préparation d'un transcrit à partir d'un échantillon provenant d'un sujet ;
   une étape de préparation d'ADNc à partir du transcrit ; une étape de concentration d'ADNc cible hybridé à un ensemble de sondes de capture ;
   une étape de soumission d'ADNc cible concentré à un séquençage par séquençage massivement parallèle ; et
   une étape de détermination de la présence ou du niveau d'expression d'un transcrit résultant d'un saut d'exon ;
   dans lequel
   le transcrit comprend l'exon A' sur le côté 5' et l'exon B' sur le côté 3' reliés l'un à l'autre à un point de jonction potentiel,
   l'ensemble de sondes de capture comprend au moins deux sondes de capture différentes qui s'hybrident à une région dérivée de l'un ou l'autre parmi l'exon A' ou B' de l'ADNc préparé à partir du transcrit, et
   $z \geq x + y$ est satisfait, où x représente la longueur nucléotidique minimale d'une extrémité de chacune des sondes de capture hybridées à l'ADNc jusqu'au point de jonction potentiel, y représente la longueur nucléotidique de la région de chacune des sondes de capture hybridées à l'ADNc, et z représente la longueur d'une lecture de séquençage massivement parallèle.

2. Procédé selon la revendication 1, dans lequel z est 50 à 500.

3. Procédé selon la revendication 1, dans lequel x représente 0 à 140, y représente 30 à 140 et z représente 100 à 300.

4. Procédé selon l'une quelconque des revendications 1 à 3, dans lequel l'ensemble de sondes de capture est un ensemble de sondes de combinaison comprenant les ensembles de sondes de capture définis dans l'une quelconque des revendications 1 à 3.

5. Procédé selon l'une quelconque des revendications 1 à 4, dans lequel l'ensemble de sondes de capture tel que défini dans l'une quelconque des revendications 1 à 3 ou l'ensemble de sondes de combinaison tel que défini dans la revendication 4 comprend en outre au moins une sonde pour mesurer le niveau d'expression génique.

6. Procédé pour déterminer la présence ou l'absence d'une maladie ou d'un risque de celle-ci, identifier le type de cancer, ou déterminer le pronostic d'un cancer, chez un sujet, comprenant
une étape de détermination de la présence ou du niveau d'expression d'un transcrit comprenant un transcrit résultant d'un saut d'exon selon le procédé selon l'une quelconque des revendications 1 à 5.

Fig. 1

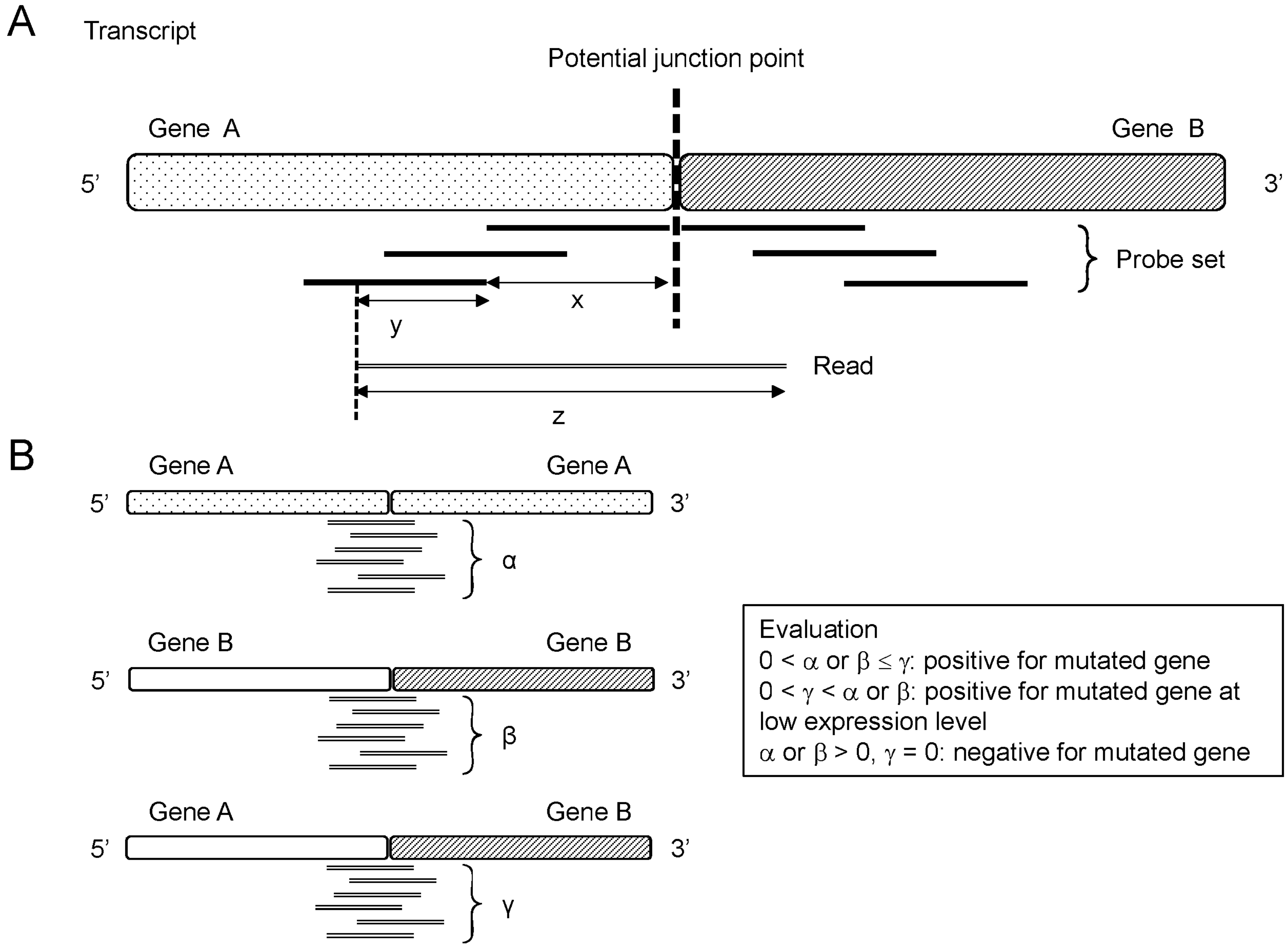
A
Transcript
Potential junction point
Gene A
Gene B
5'
3'
Probe set
x
y
Read
z
B
Gene A
Gene A
5'
3'
α
Gene B
Gene B
5'
3'
β
Gene A
Gene B
5'
3'
γ
Evaluation
0 < α or β ≤ γ: positive for mutated gene
0 < γ < α or β: positive for mutated gene at low expression level
α or β > 0, γ = 0: negative for mutated gene

Fig. 2

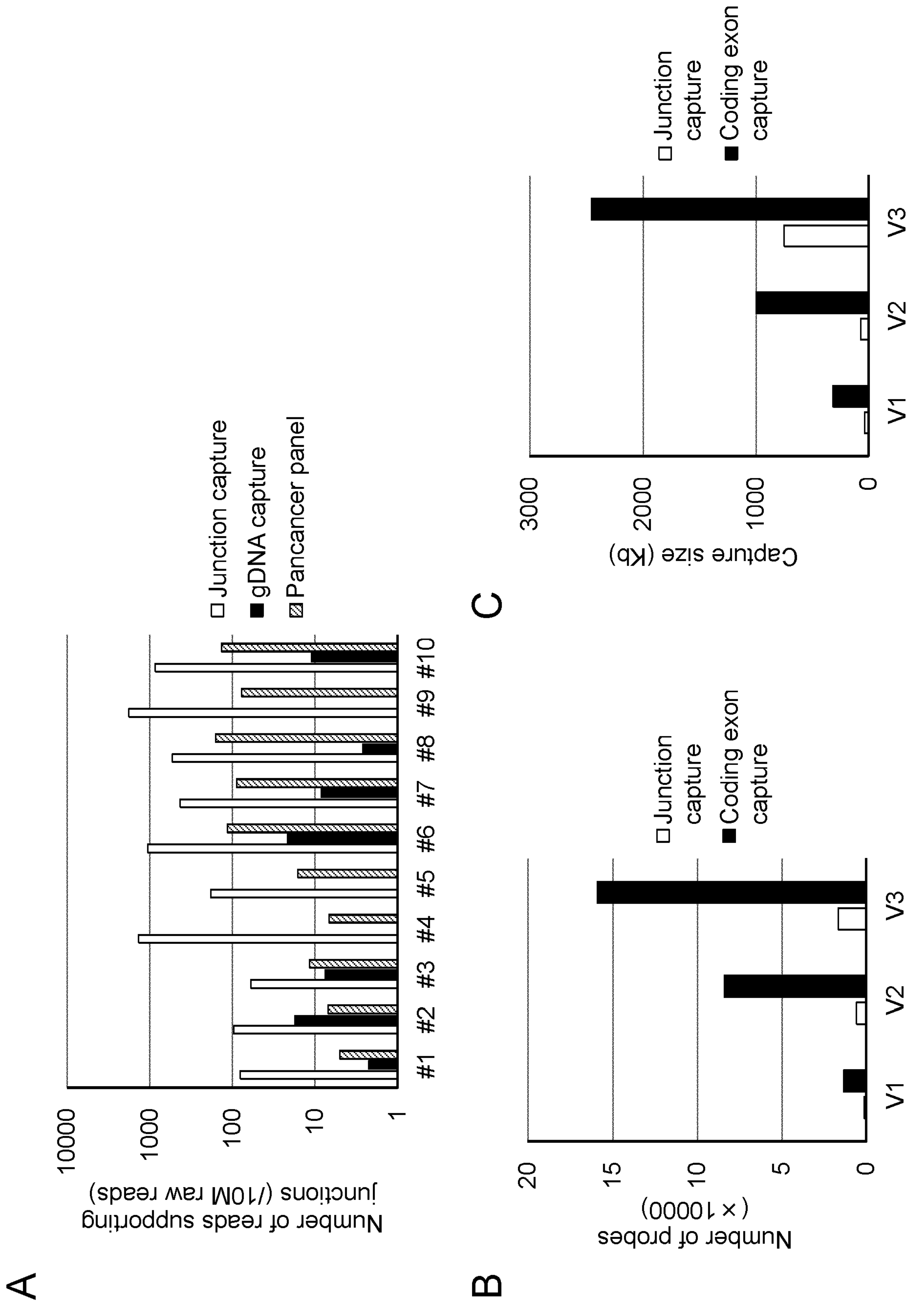
A
Number of reads supporting junctions (/10M raw reads)
10000
1000
100
10
1
#1
#2
#3
#4
#5
#6
#7
#8
#9
#10
Junction capture
gDNA capture
Pancancer panel
B
Number of probes (× 10000)
20
15
10
5
0
V1
V2
V3
Junction capture
Coding exon capture
C
Capture size (Kb)
3000
2000
1000
0
V1
V2
V3
Junction capture
Coding exon capture

Fig. 3

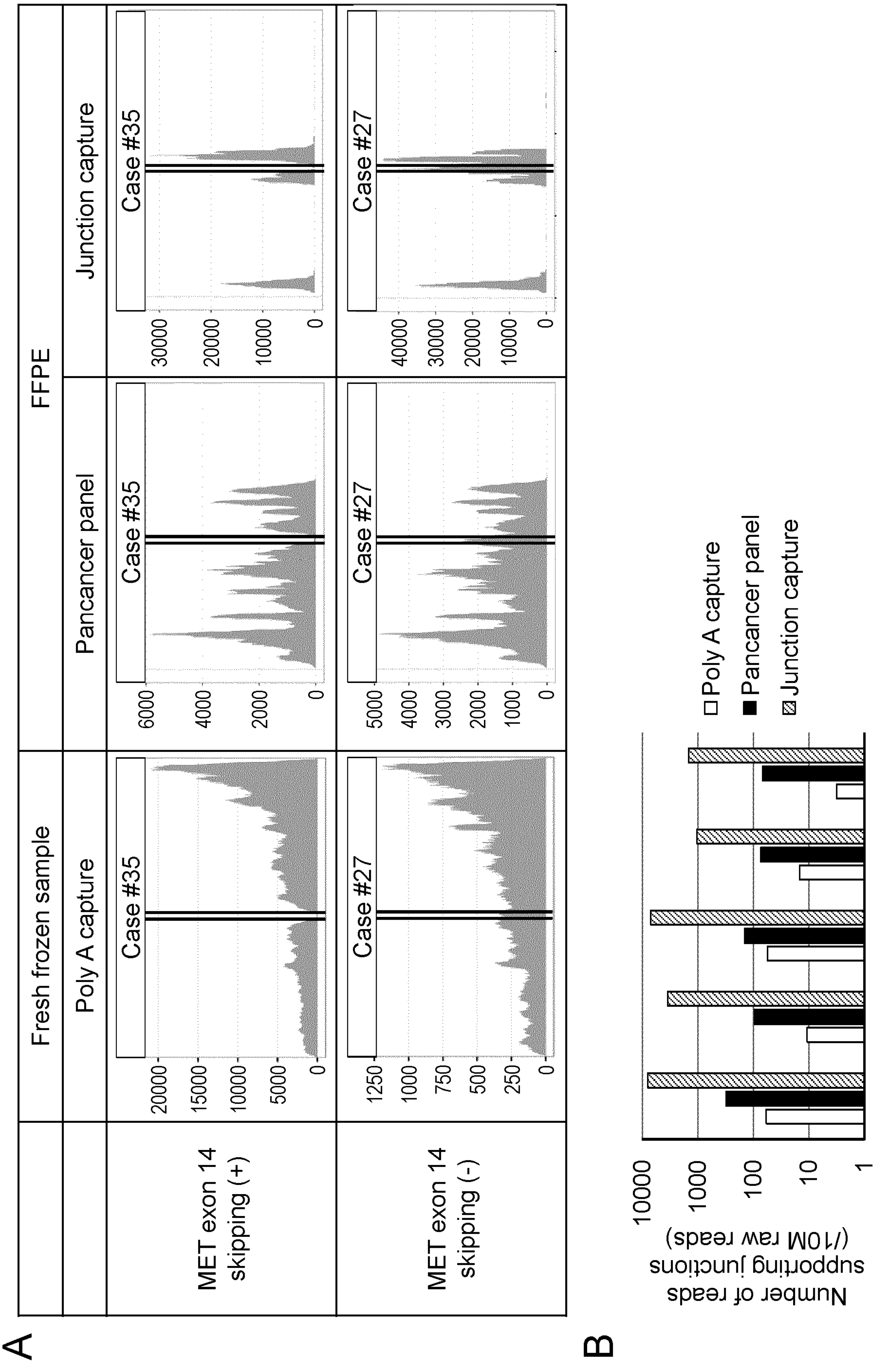

A
Fresh frozen sample
FFPE
Poly A capture
Pancancer panel
Junction capture
MET exon 14 skipping (+)
MET exon 14 skipping (-)
Case #35
Case #27
20000
15000
10000
5000
0
1250
1000
750
500
250
6000
4000
2000
5000
3000
1000
30000
40000
B
Number of reads supporting junctions (/10M raw reads)
10000
1000
100
10
1
#35
#36
#37
#38
#39
Poly A capture
Pancancer panel
Junction capture

Fig. 4

A

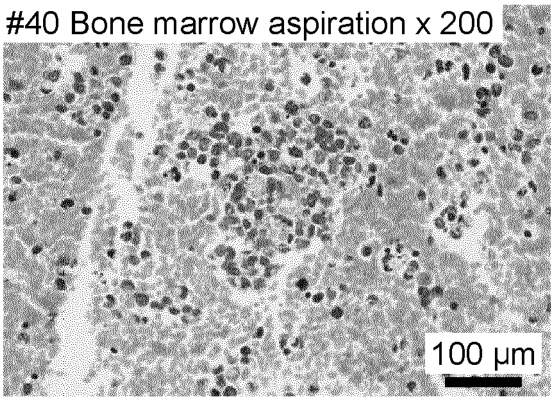


B

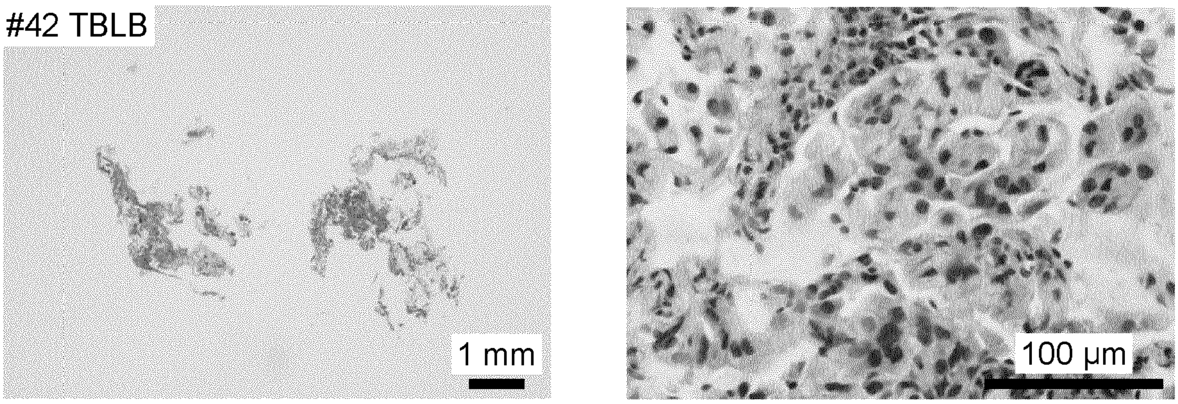

Fig. 5

A

B

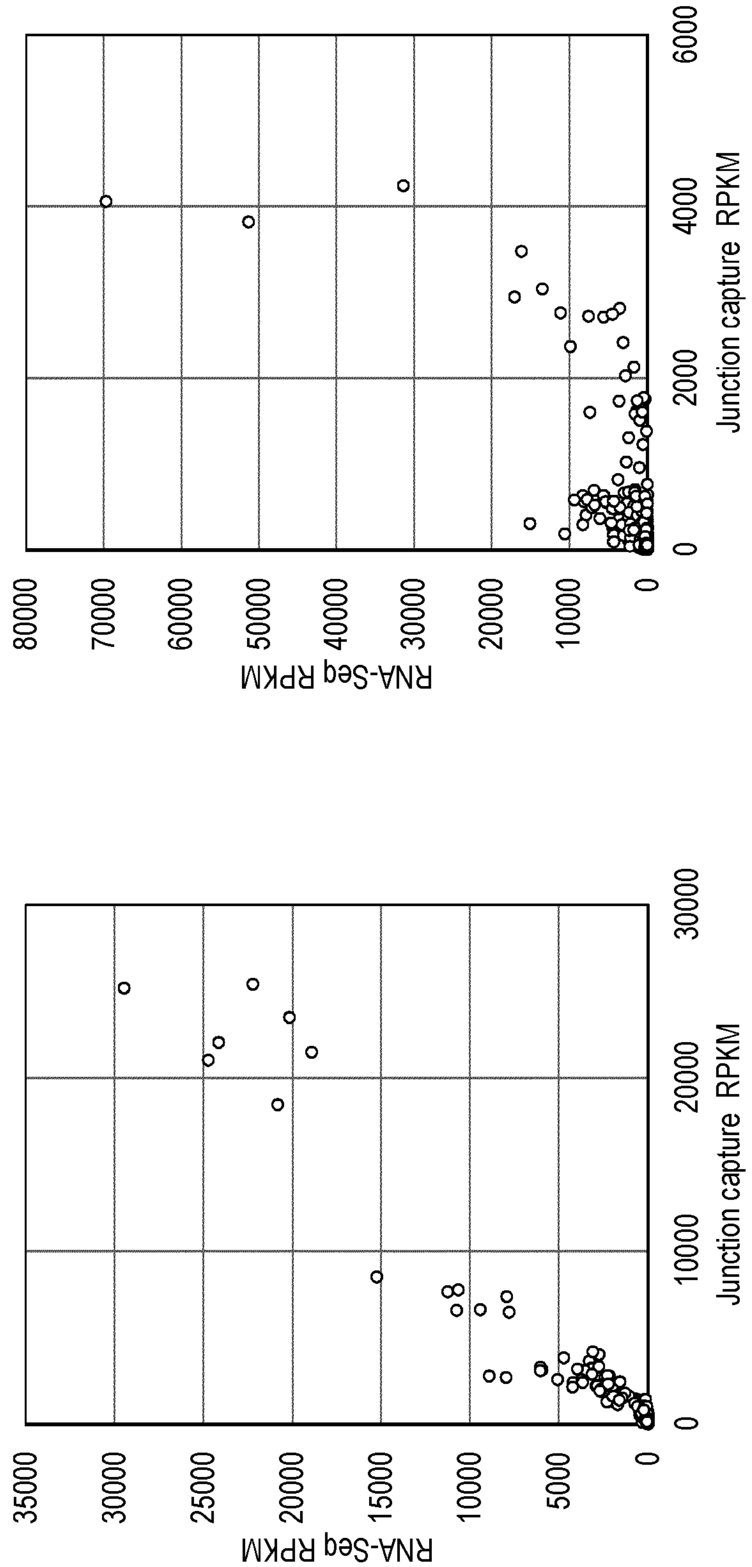
RNA-Seq RPKM
Junction capture RPKM
0
5000
10000
15000
20000
25000
30000
35000
0
10000
20000
30000
RNA-Seq RPKM
Junction capture RPKM
0
10000
20000
30000
40000
50000
60000
70000
80000
0
2000
4000
6000

Fig. 6

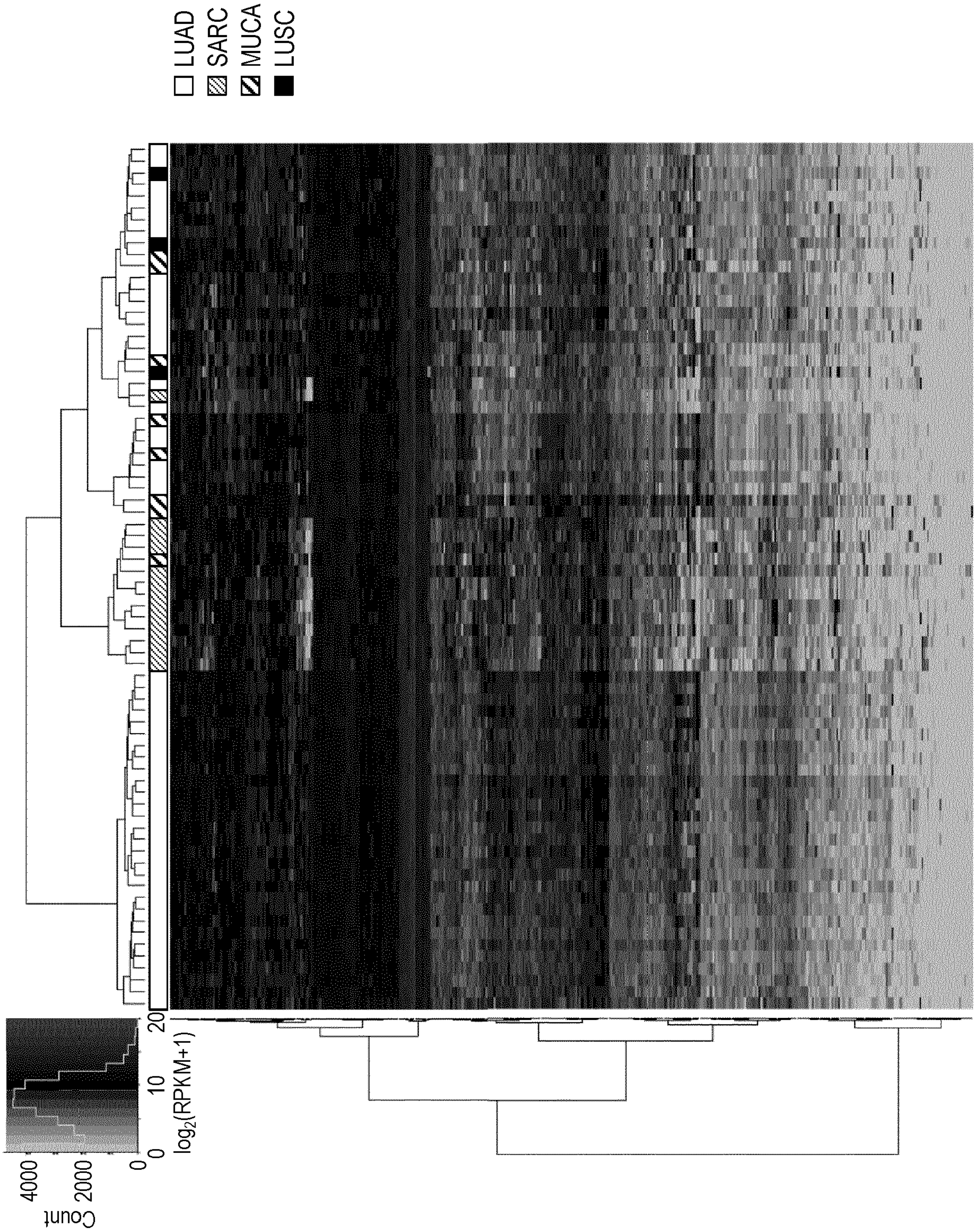

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012116248 A
- JP 2017125074 A


### Non-patent literature cited in the description


- **LEVIN et al.** *Genome Biology*, 2009, vol. 10, R115
- **J. ERIKSON et al.** *Proc. Natl. Acad. Sci., USA*, 1986, vol. 83, 1807-1811
- **M. SODA et al.** *Nature*, 2007, vol. 448, 561-566
- **T. KOHNO et al.** *Nat. Med.*, 2012, vol. 18, 375-377
- **K. TAKEUCHI et al.** *Nat. Med.*, 2012, vol. 18, 378-381
- **D. LIPSON et al.** *Nat. Med.*, 2012, vol. 18, 382-384
- **L. FERNANDEZ-CUESTA et al.** *Cancer Discov.*, 2014, vol. 4, 415-422
- **A. VAISHNAVI et al.** *Nat. Med.*, 2013, vol. 19, 1469-1472
- **R. WANG, L et al.** *Clin. Cancer Res.*, 2014, vol. 20, 4107-4114
- **RICK KAMPS et al.** *Int. J. Mol. Sci.*, 2017, vol. 18 (2), 308
- *Int. Neurourol. J.*, 2016, vol. 20 (2), 76-83
- **ALTSCHUL et al.** *Nuc. Acids. Res.*, 1977, vol. 25, 3389-3402
- **ALTSCHUL et al.** *J. Mol. Biol.*, 1990, vol. 215, 403-410
- **GREEN** ; **SAMBROOK**. Molecular Cloning. Cold Spring Harbor Laboratory Press, 2012
- **RYAN TEWHEY et al.** *Genome Biology*, 2009, vol. 10, R116

SEQUENCE LISTING

<110> The University of Tokyo

<120> PROBE AND METHOD FOR DETECTING TRANSCRIPT RESULTING FROM FUSION GENE AND/OR EXON SKIPPING

<130> P119328EP

<150> JP 2017-125074<151> 2017-06-27

<160> 12

<170> PatentIn version 3.5

<210> 1
<211> 136
<212> DNA
<213> Homo sapiens

<400> 1

aaatatgaaa agccaaaatt tgtgcagtgt ttagcattct tggggaatgg agatgttctt 60

actggagact caggtggagt catgcttata tggagcaaaa ctactgtaga gcccacacct 120

gggaaaggac ctaaag 136

<210> 2

<211> 120
<212> DNA
<213> Artificial

<220>
<223> probe

<400> 2

aatttgtgca gtgtttagca ttcttgggga atggagatgt tcttactgga gactcaggtg 60

gagtcatgct tatatggagc aaaactactg tagagcccac acctgggaaa ggacctaaag 120

<210> 3
<211> 120
<212> DNA
<213> Artificial

<220>
<223> probe

<400> 3

aatatgaaaa gccaaaattt gtgcagtgtt tagcattctt ggggaatgga gatgttctta 60

ctggagactc aggtggagtc atgcttatat ggagcaaaac tactgtagag cccacacctg 120

<210> 4
<211> 120
<212> DNA
<213> Artificial

<220>
<223> probe

<400> 4

agggaatttt tgggaaatat gaaaagccaa aatttgtgca gtgtttagca ttcttgggga 60

atggagatgt tcttactgga gactcaggtg gagtcatgct tatatggagc aaaactactg 120

<210> 5
<211> 120
<212> DNA
<213> Artificial

<220>
<223> probe

<400> 5

cactaacaag aaaacaggga atttttggga aatatgaaaa gccaaaattt gtgcagtgtt 60

tagcattctt ggggaatgga gatgttctta ctggagactc aggtggagtc atgcttatat 120

<210> 6
<211> 120
<212> DNA
<213> Artificial

<220>
<223> probe

<400> 6

cctggagcgg caattcacta acaagaaaac agggaatttt tgggaaatat gaaaagccaa 60

aatttgtgca gtgtttagca ttcttgggga atggagatgt tcttactgga gactcaggtg 120

<210> 7
<211> 187
<212> DNA
<213> Homo sapiens

<400> 7

tgtaccgccg gaagcaccag gagctgcaag ccatgcagat ggagctgcag agccctgagt 60

acaagctgag caagctccgc acctcgacca tcatgaccga ctacaacccc aactactgct 120

ttgctggcaa gacctcctcc atcagtgacc tgaaggaggt gccgcggaaa aacatcaccc 180

tcattcg 187

<210> 8
<211> 120
<212> DNA
<213> Artificial

<220>
<223> probe

<400> 8

tgtaccgccg gaagcaccag gagctgcaag ccatgcagat ggagctgcag agccctgagt 60

acaagctgag caagctccgc acctcgacca tcatgaccga ctacaacccc aactactgct 120

<210> 9
<211> 120
<212> DNA
<213> Artificial

<220>

<223> probe

<400> 9

caccaggagc tgcaagccat gcagatggag ctgcagagcc ctgagtacaa gctgagcaag 60

ctccgcacct cgaccatcat gaccgactac aaccccaact actgctttgc tggcaagacc 120

<210> 10
<211> 120
<212> DNA
<213> Artificial

<220>
<223> probe

<400> 10

gccatgcaga tggagctgca gagccctgag tacaagctga gcaagctccg cacctcgacc 60

atcatgaccg actacaaccc caactactgc tttgctggca agacctcctc catcagtgac 120

<210> 11
<211> 120
<212> DNA
<213> Artificial

<220>
<223> probe

<400> 11

ctgcagagcc ctgagtacaa gctgagcaag ctccgcacct cgaccatcat gaccgactac 60

aaccccaact actgctttgc tggcaagacc tcctccatca gtgacctgaa ggaggtgccg 120

<210> 12
<211> 120
<212> DNA
<213> Artificial

<220>
<223> probe

<400> 12

gtacaagctg agcaagctcc gcacctcgac catcatgacc gactacaacc ccaactactg 60

ctttgctggc aagacctcct ccatcagtga cctgaaggag gtgccgcgga aaaacatcac 120